(19) Europäisches Patentamt
European Patent Office
Office européen des brevets

(11) Publication number: **0 031 145**
**B1**

(12) # EUROPEAN PATENT SPECIFICATION

(45) Date of publication of patent specification: **16.01.85**

(51) Int. Cl.⁴: **G 01 N 25/22**

(21) Application number: **80108010.2**

(22) Date of filing: **18.12.80**

(54) **Method and apparatus for determining the volumetric heat content of gaseous fuels.**

(30) Priority: **20.12.79 US 105794**

(43) Date of publication of application:
**01.07.81 Bulletin 81/26**

(45) Publication of the grant of the patent:
**16.01.85 Bulletin 85/03**

(84) Designated Contracting States:
**DE FR GB IT**

(56) References cited:
**DE-A-2 815 141**
**GB-A-1 389 463**
**US-A-1 686 751**
**US-A-3 777 562**
**US-A-4 125 018**
**US-A-4 125 123**

(73) Proprietor: **HONEYWELL INC.**
**Honeywell Plaza**
**Minneapolis Minnesota 55408 (US)**

(72) Inventor: **Kude, William B.**
**10225 - 39th Avenue North**
**Plymouth, MN 55441 (US)**
Inventor: **Youngbauer, Daniel L.**
**495 Glendale Street**
**St. Paul, MN 55104 (US)**
Inventor: **Pearman, A Noel J.**
**2087 Bayard Avenue**
**St. Paul, MN 55116 (GB)**

(74) Representative: **Rentzsch, Heinz et al**
**Honeywell Europe S.A. Holding KG**
**Kaiserleistrasse 55 Postfach 184**
**D-6050 Offenbach am Main (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates generally to the field of measuring and monitoring the heating value of gaseous fuels such as natural gas and, more particularly, to an apparatus for determining the volumetric heating content or calorific value of gaseous fuels as described in the general portion of claim 1. The volumetric heat content of gaseous fuels such as natural gas are frequently given assumed average numbers. Thus, the volumetric heat content of natural gas, for example, is frequently assumed to be 1000 British thermal units (BTU's) per cubic foot, whereat 1BTU=1055,06J and 1cu ft=0,0283m$^3$ and therewith 1BTU/cu ft=37.281,272 J/m$^3$. In the past, pricing of such fuels has been based upon either the assumption of a nominal average value or the periodic checking of the actual value by a variety of time-consuming methods.

In one method chromatographic analysis of the constituents has been used to compute the actual heating value or BTU content of a given natural gas from the percentage composition of the mixture. In another method, the heat content has been determined by measuring the amount of heat liberated in burning exactly one cubic foot (2,83·10$^4$ cm$^3$) of gas (saturated with water) at standard conditions of temperature and pressure. The heat so liberated is absorbed by a weighed amount of water and the subsequent temperature rise of the water used to calculate the "gross or (higher) heating value.". This "calorimeter bomb" approach, like the chromatographic approach, has several drawbacks.

Both methods involve reasonably expensive instrumentation and require considerable labor to perform the measurements and calculations. Such testing also, of necessity, introduces considerable time delay and certainly appears less desirable than an on-line system. Even so, these might be sufficient methods if, in fact, the composition of the gas being used did not vary greatly with time. However, the composition of natural gas, for example, may vary greatly in composition depending on the gas field from which it came and the treatment it receives before distribution. The gas that reaches the customer or consumer is frequently only about 85 percent methane with the remaining 15 percent being a mixture of various hydrocarbon molecules such as ethane, propane, n-butane, i-butane, etc. Also, as much as 25 percent of the gas reaching the customer may be made up of non-combustible constituents which occur naturally or have been added to the mixture. These include nitrogen, air, and carbon dioxide. Natural gas is used herein as a representative example because it is by far the most widely used gaseous fuel.

The inerts, of course, add nothing to the volumetric heat content, and the heating value of alkane and other hydrocarbons of a higher order than methane have a higher heating value on a volumetric basis because of their higher molecular weights. In view of the great variation in constituents of natural gas, the volumetric heat content even in a single distribution system may vary greatly with time.

In addition to the other variations, numerous gas utilities have found that in severe winter weather it is cost justified to add a mixture of propane and air to the fuel in order to meet peak demand. They have found that the increased cost of propane at such peak load periods is less than the cost of additional distribution capacity which would otherwise be necessary to meet the peak loads with gas that cannot be stored as liquid adjacent to users.

As a result of all these factors, a random sampling of the heat content of the natural gas being distributed, might lead to great inaccuracies as to the actual heating value of the fuel delivered. Thus, it is necessary for the heat content of the natural gas to be continually monitured and adjusted in order to stay within promised specified limits and to assure that the user is charged for the proper amount of heating value he receives from the fuel.

Other incentives are involved in the desirability for providing an efficient on-line device for monitoring the volumetric heat content of gas. Users, especially those in industries, which require large amounts of gas for heat processing equipment can utilize such data to adjust gas input modes to provide a more uniform total heat input. They can also utilize such information to adjust burner controls so as to provide the proper air-fuel ratio and thereby avoid inefficiencies which occur when the burners are operating at a ratio which is either too lean or too rich.

Attempts have been made to provide "on-line" devices. In the prior art it is generally known that the heat content of a gaseous fuel such as natural gas is related to the ratio of fuel to air necessary for complete combustion of the gas. One prior art device utilizes a system in which the heat content of the fuel is related mathematically to that ratio of air to fuel which maximizes the adiabatic flame temperature of the mixture. In that system the air and fuel are split between two burners in such a way that the mixture in one burner has a slightly higher air-fuel ratio than the other. The air flow is allowed to remain constant, and the fuel flow is varied in the two burners until the temperature of both flames is the same. In this manner, one burner approaches the maximum flame temperature from the "rich" side and the other from the "lean" side. The fuel flow is metered as by a turbine meter, and, based on the metered rates of air and fuel, a BTU heat content is calculated assuming the maximum adiabatic flame teperature occurs at the stoichiometric point of the combustion mixture. Actually, the maximum flame temperature may be reached when the

mixture is somewhat lean, i.e. when an amount of excess air is present.

A prior art method of calculating the heat content from such measurements is found in "New Approach to the Continuous Measurement of Calorific Values of Gaseous Fuels" by William H. Clingman, Jr., *AGA Operating Section* (1972).

This method is also described in connection with figure 3 of US—A—37 77 562. That system has the drawback that it requires both accurate fuel measurement and temperature measurement of two complete burner systems for comparison.

US—A—37 77 562 in figure 1 further shows an apparatus as described in the general portion of claim 1, whereat a single burner and a single temperature sensor are used. An adjustable valve and a flow meter are connected in series in the gas stream and a control valve together with a further flow meter is connected in series in the air piping. A thermocouple measures the temperature of the flame of a burner which is supplied with a mixture of gas and air as supplied via those two valves and flow meters. A controller connected to the output of the thermocouple controls the position of the air valve such that the flame temperature is held at its maximum value. Again under the assumption that the maximum flame temperature characterizes a critical air to gas ratio leading to stoichiometric combustion or complete oxidation of the fuel, respectively, the output signals of the two flow meters are fed to a divider circuit which generates an electrical signal indicative of the critical combustion ratio. From this signal the volumetric heat content of the fuel can be calculated by using a known relationship between that critical ratio and the volumetric heat content of the constituents of said fuel. Also in this case the separate measurement of the gas and the air flow together with the finding of the point of complete or predetermined oxidation by measuring the flame temperature leads to inaccuracies which are to be overcome by the present invention.

The use of electrochemical cells as oxygen or combustible sensors to sense the residual products of combustion including electrochemical cells based on ceramic compounds such as $ZrO_2$ is also known, see US—A—41 29 099. Under normal conditions, natural gas is burned with an excess of oxygen to assure complete combustion and an absence of carbon monoxide in the products of combustion. This leads to the presence of an amount of excess oxygen after combustion which raises the possibility that an oxygen sensor based on Zirconia or the like could provide a relatively inexpensive and rapid solution to the problem of determining heating values for natural gas mixtures based on the air-fuel ratio. Such solid electrolyte-based oxygen sensors have been used to rapidly provide information for such systems as catalytic automobile exhaust control. Several different types of solid electrolyte-based oxygen sensors are commercially available.

Thus it has been proposed to use a ceramic based electrochemical made chiefly of zirconia ($ZrO_2$) which is known to exhibit a Nernstian voltage output when exposed to differing partial pressures of oxygen on each side of the ceramic material. This can be used to sense the amount of oxygen present in the products of combustion. That system proposes to utilize a known mass ratio between the fuel and oxygen supplied to a burner in conjunction with the measurement of excess oxygen after combustion using the Nernstian relationship to provide a basis for deriving the heat content of the fuel. According to that system, measurements should be made when the combustion mixture contains about 20 percent excess air, that is, not stoichiometric combustion.

That system requires that the air and fuel be controlled so as to be measured in relation to standard temperature and pressure and precise metering of the mass flows of fuel and air. Also the sensor output is decidedly temperature sensitive in the presence of more than minute amounts of oxygen. Thus, when operating in the oxygen-rich portion of the $ZrO_2$ electrochemical response curve, the temperature of the sensor must be carefully controlled. Unfortunately, the system does not take into consideration the fact that as the heating value of the gas to be measured increases, so does the average molecular weight of the gas. While this could be overcome with appropriate correction factors, if only combustible gases were involved, the molecular weight of the fuel species can also be varied because of the presence of inert species in the mixture which leads to calculation errors.

## Summary of the invention

It is the main object of the invention to disclose a highly accurate and reliable apparatus for determining the volumetric heat content of a gaseous fuel which is suitable for on-line measuring the heating content or calorific value. This is achieved by the invention as characterized in claim 1. It utilizes a solid state sensor for determining the critical air to fuel ratio where a predetermined oxidation and, in particular, a complete oxidation of the fuel is achieved. Preferably a ceramic electrochemical oxygen sensor is used. It eliminates both the need for precise temperature control of the sensor as well as errors introduced by inert constituents. The measuring apparatus further includes an accurate volumetric measuring system for simultaneously proportioning the fuel and air for obtaining a mixture, the volumetric air to fuel ratio of which can easily be determined from the operation condition of the proportioning system, in particular from its

speed of rotation. Oxidation of the fuel can be accomplished by a single sample burner which in combination with the sensor provides the necessary information about the air to fuel ratio and, in particular, for controlling the mixing device such that this ratio is adjusted to a critical or stoichiometric ratio where the fuel is completely oxidized without any excess oxygen in the exhaust gases.

Preferred embodiments of the invention are described in the subclaims. Where grooves are provided in the stationary or rotating parts of the mixing device for connecting the proportioning volumes to the inlet and outlet junctions, it is apparent that such grooves can either be machined in the surface of the groove-carrying part or can be formed by slots extending through the entire thickness of the plate and closed on one side by a sealing or cover.

The system is designated to control oxidation or combustion at the stoichiometric point, wherein the electrochemical sensor exhibits a step-change function to produce increased accuracy. At this point, the precise volumetric ratio of fuel to air is accurately known from the measuring system and the heat content of the fuel can be accurately determined from that ratio in a manner simplified by the elimination of the effects of several undesirable variables.

The fuel heat content metering system of the present invention includes a precise, adjustable metering system which accurately proportions an amount of fuel gas or calibration gas to be tested with a known amount of air such that at any given time, the volumetric ratio of air, and therefore, to fuel is precisely known. The mixture is fed to a burner system in which the fuel is combusted in the presence of a solid state ceramic electrochemical cell which provides a step-change in voltage output as the amount of residual oxygen or combustibles approaches zero, that is, approaches the point of stoichiometry. An electrical signal from the electrochemical cell is utilized with a programmable electronic processing system to adjust the fuel mixture in accordance with the output of the electrochemical cell to achieve and determine the stoichiometric air-fuel ratio as signalled by the rapid change in electrical output of the cell at that point. The air-fuel ratio at that point is known from the measuring system and the heat content of the fuel is readily determined therefrom.

In the preferred embodiment, the metering of the fuel and air is accomplished by one of several alternate, novel systems which provide precise, time proportioning volumetric measurement. In one embodiment of the proportioning system of the invention, fuel and air are fed through a rotary valve system in which the proportion of air or gas transferred therethrough to the combustion chamber depends on the speed of rotation of the rotary valve. The rotating member contains hollow chambers which are alternately filled with fuel and purged with air as they are rotated in the fashion of a Gatling gun. The air flow remains constant and the fuel introduced varies with the angular speed of the rotor. A motor, the speed of which can accurately be controlled, as with a stepping motor, is provided to allow for a great deal of flexibility in adjusting the rotation speed and thus the air-fuel ratio.

Inasmuch as fuel and air are fed to the measuring burner at the same regulated pressure and, if packaged together, will be at or close to the same temperature, thus eliminates the additional variables relating to differences in temperature and pressure which otherwise would have to be compensated by measuring the temperature and pressure and adjusting the calculation therefor. Temperature and pressure compensation, of course, may be provided in the fuel and air supply for applications wherein the keeping of equal pressure and temperature is not practicable.

Because precise volumetric measuring is used, the need for compensating for changes in molecular weights of combustibles or for the addition of inert components is also eliminated. Calculation of heat content is made utilizing a microprocessor system from a simplified known constant relationship between the air-fuel ratio at the stoichiometric point which is known from the speed of the sampling system and the corresponding fuel heat content. The results may be recorded or displayed in any manner desired.

The heat given off by the combustion of the given mixture can also be utilized to maintain the temperature of the electrochemical cell within the desired range. In the preferred embodiment, the cell within the desired range is a ceramic cell primarily made up of $ZrO_2$ stabilized with an amount of CaO, MgO, or $Y_2O_3$, to stabilize its temperature related response such that in the step-change portion of the sensing curve, the cell is repeatably accurate without temperature compensation from about 315 to 770°C.

Brief description of the drawings

In the drawings wherein like numerals are used to designate like parts throughout the same:

Figure 1 is a perspective view of a mounted in-line instrument utilizing the invention;

Figure 2 is a schematic block diagram of the preferred embodiment of the invention;

Figure 3 is a view partially in section of a rotary valve system of the embodiment of Figure 2;

Figure 4 is a reduced, exploded view of the rotary valving system of Figure 3;

Figure 5 is an enlarged view of an alternate rotary valving system of the embodiment of Figure 2;

Figure 6 is an exploded view of the rotary valving system of Figure 5;

Figure 7 depicts the layout of the multi-sheet Figures 8, 9, 11 and 12.

Figure 8 is an electrical block diagram of the embodiment of Figure 2.

Figure 9 is a diagram of electrical details of the diagram of Figure 8.

Figure 10 is a block diagram of the logic diagrams of Figures 11—16;

Figure 11 is a logic diagram for the main routine system of Figure 10;

Figure 12 is a logic diagram for the startup task of Figure 10;

Figure 13 is a logic diagram for the search task of Figure 10;

Figure 14 is a logic diagram for the calibrate task of Figure 10; and

Figure 15 is a logic diagram of the display task of Figure 10;

Figure 16 is a logic diagram of the read temperature task of Figure 10;

Figure 17 is a represenstative curve of the response in millivolts versus the air fuel ratio of a typical $ZrO_2$ sensor in accordance with the invention.

Figure 18 is a plot showing heat content of various hydrocarbon fuels.

Description of the preferred embodiment

The heat content measuring system of the present invention contemplates a simple, accurate method and apparatus for determining the precise ratio of air to fuel in a combustible mixture which can be utilized, in turn, to accurately determine the heat content of the fuel used. This system does not require remote sampling and can actually be used in an on-line manner. As will be seen from the detailed description, substantially all of the sources of error introduced in previous attempts to achieve accurate on-line readings have been substantially eliminated.

In Figure 1, there is shown generally at 20 an in situ placement of a heat content measuring device in accordance with the present invention. Thus, the device can be mounted on a fuel line 21 and connected to it as by a sample line 22 such that continual on-line measuring of the fuel passing through the line 22 can be achieved. As depicted in Figure 1, the apparatus may have an explosion proof case having halves 23 and 24 securely bolted together. Venting is provided as at 25. Typical adjustment knobs 26 and 27 corresponding to display readouts 28 and 29 are represented which may read out directly in air-fuel ratio.

Figures 2 and 3 depict alternate embodiments of the heat content measuring system of the invention. The system of Figure 2 includes an air supply system, both calibration and unknown or sample gas supply systems, a mixing system or proportioning system, and a combustion and measuring system.

The preferred embodiment is shown in Figure 2. This includes an inlet sample fuel gas line 30 which includes corresponding filter 31, pressure regulator 32, and orifice 33, and, similarly, for the calibration gas system there is an inlet line 34, filter 35, regulator 36, and metering orifice 37. The lines 30 and 34 converge upon a three-way valve 38. Similarly, air is provided in line 39 by blower 40 with filter 41, associated pressure regulator 42 and metering orifice 43. A sample or main combustion chamber is provided at 44 with burner 45, flame safeguard unit 46 with associated flame igniter-sensor 47 and the electrochemical combustion product sensor 48. Contact 49 associated with the main safety shutoff valve 50 is provided to shut off the fuel flow should a flame out or other problem occur. Similarly, an auxiliary contact 51 is provided for external use, also controlled by the main burner flame safeguard system 46. A flare and vent system is provided with combustion chamber 52, burner 53, flame sageguard unit 54 with its associated igniter-sensor 55. The main and flare combustion chambers are connected to their associated flame safeguard systems by conductors 56 and 57 respectively. The lines 56 and 57 also represent 16 conductor for the spark ignition in this schematic drawing.

Additional components include an air temperature sensor $T_1$ and fuel mixture temperature sensor $T_2$ with respective associated leads 58 and 59.

The embodiment of Figure 2 introduces a completely distinct and unique proportioning and mixing concept including a rotary mixing valve 60 driven by an associated motor 61. The rotary valve is supplied by the gas of interest by line 62, and air by line 63. The proportional mixture is conveyed to the main burner by 64 and residual fuel to the vent burner by 65. The operation and details of the mixing system 60 and associated motor 61 will be described in greater detail with reference to the alternate embodiments thereof shown in Figures 3—8.

The main electrical components which will be described below are shown in block form in Figure 2 and include a conventional microprocessor control board 70. Temperature information of lines 58 and 59 is processed by an analog-to-digital converter 71 and supplied to the control board by 72. Flame safeguard information is received on lines 73 and 74 and information from the sensor 48 on line 75 via a threshold detector 76. Various other inputs are received at 77. A display 78, alarm 79, and printer 80 receive information from the board 70 along respective routes 81, 82, and 83.

In Figure 3, there is pictured one embodiment of a rotary valve proportioning system in accordance with 60 of Figure 2. The internal structure of the system is depicted in greater detail in the exploded view of Figure 4. The basic components of the rotary valve system include the motor 61, the proportioning distribution rotor 100 flanked by a distribution plate 101 and an end plate 102. The shaft 103 of the rotor 100 is coupled to the shaft 104 of the

motor 61 as by a flexible coupling 105. The end plate 102 is spaced from the motor mounting face plate 106 and secured thereto as by a plurality of radially distributed bolts 107 secured as by nuts 108 and 109. As can more adequately be seen in Figure 4, the threaded bolts 107 are of such a diameter as to pass through holes 110 in the member 101 such that the plate member 101 is free to slide along the axes of bolts 107. The rotor member 100 is provided with flanking seals 111 and 112 and the entire assembly including the rotor 100, distribution plate 101, and end plate 102 with the seals 111 and 112 is held in place as by a helical spring 113 which pushes on the members 101 and 106 to hold member 101 in position at the desired pressure. The fuel inlet line 62 and air inlet line 63 are connected to the member 101 as by fitting at 114 and 115, respectively. The mixture to be combusted or vented leaves the mixing system as via fittings 116 and 117 connecting lines 64 and 65.

By means of the rotary mixing system depicted in Figures 3 and 4, air and fuel entering the system is precisely proportional in a unique manner. As seen in Figure 4, the distribution member 101 is provided with an elongated partial annular groove or slot 118 and a shorter partial annular groove or slot 119. Both slots 118 and 119 may be of square section or semicircular section as desired. (It should be noted that the single member 101 in Figure 4A has been flipped over or rotated 180° with respect to the other members of the exploded view to expose the internal slotted or grooved structure of the member). Corresponding grooves 120 and 121 are provided in the member 102. The rotor member 100 is provided with a symmetrical pattern of radially distributed holes 122 forming hollow volumes or cylinders of known volume extending through the member 100, and the seal members 111 and 112 are provided with a similar pattern of holes designated 123 and 124, respectively. Seals 111 and 112 are provided with a series of alignment holes as at 125 which fit over machined pegs as at 126 in the rotor member 100 to assure proper alignment of the seals with the rotor 100 such that the pattern of holes are aligned internally to connect the grooves 118 and 119 with the grooves 120 and 121, respectively. Inlet holes or parts 127 and 128 connect the grooves 118 and 119 with the respective air and fuel inlets at 115 and 114. Outlet holes or parts 129 and 130 connect with outlets 116 and 117, respectively in a like manner.

In this manner, the fuel in line 62 passes into the groove 119 and the air from conduit 63 passes into the groove 118. Thus, the groove 119 at all times is open to incoming fuel, and the groove 118 at all times is open to incoming air. When the motor 61 is enegized, the rotor 100 and associated seals 111 and 112 are rotated with the motor shaft speed. Thus causes the hole systems of the rotor member 100 to alternately be exposed to the fuel of groove 119 and air of groove 118. The grooves 120 and 121 with associated outlet ports 129 and 130 therein are always connected respectively to the mixture outlet lines 64 and 65.

In operation, amounts of regulated air and sample or unknown fuel gas of interest are fed to the inlet conduits 63 and 62 and the motor 61 which is preferably a stepping motor is energized through the electronic system which includes a stepping motor drive control (discussed in detail below in conjunction with the discussion with the electronic system).

As the shaft of the stepping motor rotates, the direct coupled rotor 100 of the air-fuel proportioning system also rotates at the same speed. The air groove 118 in the distribution member 101 is continuously supplied with air, the groove 119 is continuously supplied with sample. Rotation of the member 100 together with the aligned seals 111 and 112 aligns and connects each of the cylindrical passages 122 alternately with the groove 119 and the groove 118.

The members 101 and 102 are disposed in fixed alignment such that hte corresponding air groove 118 and 120, and the fuel grooves 119 and 121 face each other through the rotor 100. Thus, any given port 122 aligned with the supply groove 118, of course, connects to the outlet groove 120 such that during this time, the contents of any aligned cylinder are caused to flow into the outlet groove 120 and through the port 129, and the corresponding hole 122 is filled with air. Upon reaching the position of the aligned grooves 119 and 121, the port is then exposed to the sample fuel, and the air is expelled or pushed out through the port 130 and the cylinder 122 filled with the sample fuel such that when the angular displacement of the particular cylinder 122 again aligns with the grooves 118 and 120, the fuel will be displaced by air with the fuel being dispensed through the port 129.

As can be seen in Figure 4 the annular air grooves 118 and 120 are much longer than fuel grooves 119 and 121. Nominally the air grooves may encompass about 240° and the fuel grooves 60° with two 30° spaces therebetween. This allows for the continual passage of an amount of air through the system. It can also be readily seen that the ratio of fuel to air reaching the groove 120 and passing through the outlet port 129 and eventually the burner 45 (Figure 2) will be directly proportional to the angular speed of the rotor 100. The faster that the rotor rotates, the higher the proportion of fuel to air that will be passed into the main combustion system.

So far as the mixture vented through the opening 130 through conduit 65 is concerned, the theory of "plug" flow would dictate that since the fuel which had been picked up by any given cylindrical volume chamber 122 would

have been exhausted at the groove 120, only air would be exhausted through the vent at 65. In reality, however, because of some diffusion mixing, the gas is not entirely purged from each of the cylindrical volumes 122 of the rotor 100 such that a minute amount of residual fuel is expelled in the mixture at outlet opening 130. This adds to the overflow of the supply gas which is sufficient to sustain the small vent burner or flare 53. If necessary an auxiliary supply line (not shown) may be provided from the sampling system inlet line 62 in a well known manner to sustain the flame in the burner 53.

The inlet and outlet ports connecting the grooves are preferably located at the extremes of the grooves in either embodiment. In the case of the fuel system, the inlet of the shorter groove should be at the point last encountered by the rotor and the effluent exit should be at the end of the groove or slot first encountered. In this manner, the fuel tends to flow through the supply slot and the effluent through the exit slot in a direction opposite to the direction of rotation of the rotor member. The inlet and outlet accesses for the air and mixture of interest, the inlet should be at the extreme first encountered by the rotor and the mixture outlet at the extreme last encountered by the rotor such that the air tends to flow in the same direction as the direction of the rotor rotation. This enhances the operation of the system to achieve improved purging and mixing.

The important point is the fact that equilibrium is soon reached and precise repeatable desired ratios of fuel to air can be obtained through the outlet opening 129 such that the mixture at the main burner 45 may be precisely controlled in accordance with the speed of the rotor 100. This, in fact, is accomplished with a great degree of accuracy. The air, of course, continually flows through the system as there are always cylindrical volumes 122 connected between the air supply and the outlet to the main burner 45 through the rotor 100.

An alternate embodiment of the rotary air-fuel proportioning system 60 is shown in Figures 5 and 6. This embodiment may be interchanged with the previously described embodiment 60 in fuel heat content measuring system of Figure 2.

In Figure 5 there is shown an elevational view of the alernate embodiment of the rotary valve, the exploded details of which are shown in Figure 6. This system includes a motor 140 similar to 61 which may have an integral base 141 with mounting holes 142, air-fuel distribution stator member 143, a rotor member 144 with associated plate 145, and a cover member 146. The stator distribution member 143 is provided with annular disposed recessed grooves or slots. These include longer slots 147 and 148 and shorter slots 149 and 150 which may be recessed in the surface in a shape rectangular or semicircular in nature as with the slots of the embodiment previously described. Four access ports are provided from the circumference 151 of the stator distribution member 143 at 152—155 which may be in the form of radially disposed drilled holes which are located below the bottom of the grooves 147—150 and connected to the respective grooves as by four holes in the base of the grooves as at 156—159. The access ports 152—155 are then respectively connected as by four inlet elbow pipe fittings 160—163 as shown in Figure 6. In this fashion, inlet fitting 160 is directly connected with to the groove 147, outlet fitting 161 to groove 148, inlet fitting 162 to groove 149, and outlet fitting 163 to the groove 150.

The rotor member 144 is provided with two concentric, symmetrically distributed series of radially disposed holes 164 aligned radially in pairs. The member 145 is provided with a series of radially disposed recesses in the form of grooves or slots 165 such that when the relative positions of the rotor 144 and member 145 are in proper alignment, each of the radial pairs of holes 164 aligns with a recessed slot 165 therewith forming a "U" tube of defined volume. The members 144 and 145 are secured together as by screws or bolts at 166.

When assembled, the hollow shaft 167 of the rotor member 144 is keyed to and receives the motor shaft 168 and is secured thereto as by set screws at 169. Access to the set screws may be provided as by an additional radial opening 170 beneath the grooves in the face of the stator distribution member 143. The unitary structure including members 144 and 145 is adapted to be received in the recess 171 of the cover member 146 such that it is free to rotate therein. However, the smallest practical clearance should be maintained between the rotating structure front face 172 and the front face 173 of the stator member 143. This assembly is bolted together as through holes 142 in motor mount 141 to stator 143, and through holes 174 in cover 146 to stator 143 to secure the assembly together with the unitary structure including combined members 144 and 145 free to rotate with the motor shaft within the assembled housing in extremely close proximity to the member 143.

In this manner, a series of radial "U" tubes are provided connecting the inner and outer grooves of the stator 143. The fittings 162 and 163 are connected to fuel in and fuel out or vent lines corresponding to lines 62 and 65 of Figure 2. Fittings 160 and 161 are respectively connected to incoming air and precisely-ratioed mixture lines as at 63 and 64 of Figure 2.

One successful embodiment utilized by 12 U-tubes symmetrically radially distributed in the combined rotor member which yields a 30° separation. The shorter slots 149 and 150 in the stator member 149 subtended an angle of 60° and the longer grooves or slot members 147 and 148 subtended an angle of about 240° of the circumference of the member 143

with 30° separating both extremes of the grooves. This, of course, means that at least two of the "U" tube members will always be aligned connecting the slots 149 and 150 and at least eight "U" tube members likewise will always connect the corresponding slots 147 and 148 at any given instant in time no matter what the relative rotation position of the combined member 144—145.

The operation of the proportioning system of this embodiment is easily described by selecting any "U" tube system and following its progression as it rotates about in the system. At some point in time, a given "U" tube will encounter the fuel section of the system connecting slots 149 and 150 such that regulated volumetric flow of fuel flows through the opening 158 filling and purging the slot 149 and the given "U" tube, also purging and filling the slot 150 and finally leaving through opening 159 and fitting 163 into the vent or flare gas line to be vented and burned at 49 in Figure 2. The fuel flow rate, highest anticipated motor speed, and "U" tube volumes are sized such that for any reasonably anticipated stoichiometric air-fuel ratio, the "U" tube will be completely filled with fuel and all the air expelled during the interval in which it connects the slots 149 and 150. Continued rotation brings the "U" tube across a 30° blank section of the stator and the metered fuel in the "U" tube remains trapped therein. As the fuel filled "U" tube is exposed to the 240° segment of slots 147 and 148, air is allowed to enter at the controlled volumetric flow rate and proceeds through the system. During this interval the U-tube connects slots 147 and 148 such that air entering the pipe fitting 160 pushes the fuel contained in the "U" tube into slot 148 and eventually out through the radial hole 153 and fitting 161. The fuel, now mixed with air, proceeds as through line 64 in Figure 2 to the sample or main combustion burner 45 where it is burned in accordance with the present invention.

From the configuration, it can be seen that eight "U" tubes are exposed to air simultaneously and that the mixture in line 64 will be a mixture of the flows from all eight "U" tubes. It can also readily be seen that the air flow supplied to the combustion chamber through the rotary proportioning system is totally independent of the rotary valve operation inasmuch as there are always the same number of U-tubes supplying air. The fuel flow rate, on the other hand, is determined by the frequency at which "U" tubes are filled and flushed by the air, that is, the rotational speed of the valve and the volume of the "U" tube. Of course, the U-tube volume is constant for any particular device. Therefore the air-fuel ratio of the burner 45 will be directly proportional to the speed of the rotary valve within the limits stated above.

The pre-mix burner which does not require secondary or atmospheric air to operate properly and which will function at the desired flow rate.

The vent gas burners may be any conventional atmospheric burner such as a Bunson burner. It has been found that during the operation of the system the burner flame will be sustained by the vented effluent gases. However, provision may be made for auxiliary fuel and air to be suplied from the system if desired. The flame sensors and flame safeguard units are highly conventional and readily available commercially as a model S-86 manufactured by a assignee of the present invention. In either embodiment, the pressure regulators may be any accurate low flow, low pressure regulators such as 912 series pressure regulators available from the Fisher Controls Company of Marshalltown, Iowa. The main burner of either embodiment is typically operated at a pressure of about 2,5 bar. w.c. and a firing rate of about 0,17$m^3$/h of mixture.

The rotary proportioning systems of Figures 3—4 and 5—6 provide accurate, perdictable means for combining two fluids in any desired proportion. The proportion can be determined simply from the rotational speed of either alternative embodiment of the rotary valve or by the switching speed of the valving system of Figure 3.

As is well known in the art, the open-circuit voltage of a simple electrochemical cell is established by a chemical potential difference which may be based on composition, concentration, pressure, etc., between the anode and the cathode compartments. The particular species to which the cell is sensitive is the same for both the anode and the cathode compartment, the open-circuit voltage should be zero. If, however, one of the electrode compartments is exposed to a reference quantity of the specie of interest and the other electrodes exposed to a medium containing a different amount of such specie, an open circuit potential difference is thereby established. Various ceramic compounds are known to establish such electrochemical cells in the presence of differing amounts of oxygen. In the preferred embodiment of the present invention, a modified compound consisting essentially of zirconium oxide ($ZrO_2$) is utilized as a ceramic-based electrochemical cell for the sensor. The electromotive force of the cell is created by exposing one of the electrodes to a medium containing a reference amount of oxygen, namely air, and the other to the products of combustion of the sample fuel of interest in the sample combustion chamber. Thus, when the oxygen concentration in the combustion chamber differs from that of the reference air, a potential difference is set up across the cell which can be measured and/or utilized in accordance with the present invention.

The $ZrO_2$ sensors of the present invention are normally stabilized with CaO, MgO, or $Y_2O_3$ which enhances the chemical and physical

stability of the zirconium oxide for use as a sensor. The typical configuration of an electrochemical sensing cell in accordance with the present invention may be represented by

$$O_2r,\ Pt/ZrO_2 \cdot CaO/Pt,\ O_2s$$

where r stands for reference and s sensed or sample. Noble metal, normally platinum electrodes are utilized in conjunction with the anode and cathode of the zirconium oxide sensor.

As can be seen in the curve of Figure 17, in the rich portion of the curve, which indicates the presence of excess combustibles, and in the lean portion of the curve (representing excess oxygen in the combusted gases), the curve does not change a great deal with respect to change in either the concentration of combustibles or oxygen but the center portion of the curve, however, which is close to the stiochiometric point or the point where the amount of oxygen present is just sufficient to oxidize the hydrocarbon components, the curve undergoes the step change function wherein the voltage output varies a great deal with very little change in oxygen concentration in the combustion chamber.

It has been proposed to use the $ZrO_2$ sensor in the lean portion of the curve to relate the partial pressure of oxygen present to the air-fuel ratio. As previously stated, as the amount of excess air increases above the stoichiometric point, a non-linear curve results. This portion of the curve has been found to be a Nernstian response region wherein the variation of the cell open-circuit voltage with the partial pressure of oxygen present at the measuring electrode is given by the well known equation:

$$E = \frac{RT}{nF} 1n\ (P_{O_{2ref}}/P_{O_{2sens}}),$$

wherein E is given in voltage units (volts) R and F are constants and T equals the absolute temperature. It can readily be seen from this equation that the voltage output varies inversely with the natural logarithm of the partial pressure of oxygen in the combustion chamber and directly with the absolute temperature. Making use of data desired in this region of the curve, of course, requires very accurate temperature and voltage measurements in order to discern the applicable air-fuel ratio. Otherwise errors are easily introduced in the results.

Unlike prior art systems which suggest measuring the residual oxygen in the Nernstian response region, the present invention contemplates utilizing the step response evident at or near the stoichiometric point in which the output of the cell varies greatly with an extremely small change in the air-fuel ratio. This rapid change is far easier to detect and more accurate utilize than are points in the Nernstian region.

In either embodiment, when the mixture of sample fuel and air is combusted in the combustion chamber, the chemical composition of the combusted gas mixture is sensed by the modified zirconium oxide sensor thereby producing an electrical response in accordance with the curve depicted in Figure 17. When the mixture in the combustion chamber is such that the products and combustion approach stoichiometry either from the rich or the lean side, an extreme step change is noticed in the output voltage. This step change can be utilized as a control signal of stoichiometry which is repeatably precise and eliminates the errors associated with gradual changes in a curve.

The use of stoichiometric point in the combustion of in the given alkane hydrocarbon fuel gas will now be more fully developed. The stoichiometric combustion equation for methane, the chief component of natural gas fuels, is given by the following equation which assumes that the air utilised for combustion comprises 20.83 percent $O_2$ and 79.17 percent $N_2$ and other inerts:

$$CH_4+2(O_2+3.76N_2)\ldots \rightarrow CO_2+2H_2O+7.5N_2$$

Therefore, wherein

$$A/F_{CH_4}$$

represents the air-fuel ratio of methane

$$A/F_{CH_4}=(2)(4.76)/1=9.52$$

Table 1 represents stoichiometric air-fuel ratios of various alkane hydrocarbons through pentane and adds a column HHV which represents the Higher or gross Heating Value of each particular specie in units of calories/liter or BTUs per cubic foot at 20°C, 76 cmHg which are derived from Marks Mechanical Engineering Handbook Seventh Edition. The HHV includes the latest heat of vaporization for steam formed in the combustion reaction. It should be noted that in deriving the actual air-fuel ratios, a value of 0 has been assigned to inerts which normally carry through the system unchanged and an arbitrary value of minus 4.76 has been assigned to each $O_2$ based on its combination with alkane hydrocarbons in the combustion reaction.

TABLE 1

| SPECIE | A/F | HHV* |
|---|---|---|
| $CH_4$ | 9.52 | 8852,8 |
| $C_2H_6$ | 16.66 | 15509,1 |
| $C_3H_8$ | 23.80 | 22072,9 |
| $C_4H_{10}$ | 30.94 | 28618,8 |
| $C_5H_{12}$ | 38.08 | 35156,8 |
| INERTS | 0 | 0 |
| $O_2$ | −4.76 | 0 |

*calories/liter at 20°C, 76 cm Hg

In the view of the above, for a sample of mixed saturated alkane hydrocarbons, inerts and oxygen, the air-fuel ratio of a sample ($A/F_s$) may be represented by

$$A/F_s=9.52x_1+16.66x_2+23.8x_3+30.94x_4+38.08x_5-4.76x_7$$

wherein the x subscript represents the fraction of that hydrocarbon constituent having the given number of carbon atoms through 5 and the 7 has been assigned to oxygen. The higher heating value of the sample ($HHV_s$) then can be given by

$$HHV_s=\Sigma_{i=1}^{5}(HHV_i)(x_i)$$

As explained above, each of the fuel proportioning systems utilized in the heat content measuring system of the present invention depends upon displacing fuel by air from a hollow cylindrical or tubular member at a specific volumetric frequency and running air through the same cylinders for a certain amount of time all at the same controlled flow, that is air. For any of these cylinders, then, the cycle time ($t_c$) can be represented by

$$t_c=t_a+t_f \qquad (I)$$

wherein $t_a$ equals the time devoted to the passage of air and $t_f$ represents the time devoted to the passage of fuel. Thus, the time devoted to fuel, $t_f$, can also be represented by

$$t_f=\frac{V_1}{q_a} \qquad (II)$$

wherein $V_i$ is the volume of any given cylinder and $q_a$ equals the volume flow rate of air. Inasmuch as all the fuel in the system is pushed through the cylinder at the air flow rate, the air and fuel flow rates of flow must be identical on a time basis. Substituting for $t_f$ in equation (II), we have

$$t_c=t_a+\frac{V_i}{q_a} \qquad (III)$$

Rearranging equation (III), gives us

$$t_a=t_c-\frac{V_i}{q_a} \qquad (IV)$$

Because of the identical flow rates as explained above, by definition

$$A/F=\frac{t_a}{t_f} \qquad (V)$$

and, by substituting for $t_a$ by equation (IV) and for $t_f$ from equation (II), we have

$$A/F=\frac{t_c-V_i/q_a}{V_i/q_a} \qquad (VI)$$

which, upon simplifying, yields

$$A/F=\frac{t_cq_a}{V_i}-1 \qquad (VII)$$

Also, as explained in conjunction with the description of the apparatus, above, the volumetric flow rate of air may be any arbitrary fixed rate established in the instrument by pressure regulation, volumes, etc., and the volume of each cylinder utilized in the particular proportioning system similarly acts as a limiting constant. Given this situation, let

$$K_1=\frac{q_a}{V_i} \qquad (VIII)$$

If we substitute $k_1$ for $q_a/V$ in equation VII, the result is

$$A/F=K_1t_c-1 \qquad (IX)$$

Returning again to Figure 18, by use of that figure, the following may be derived;

$$HHV_s=922{,}75\ A/F_s+68{,}26 \qquad (X)$$

A combination of equations (IX) and (X) thus yields

$$HHV_s=922{,}75\ k_1t_c-1+68{,}26$$

which upon simplifying yields

$$HHV_s=922{,}75\ k_1t_c-854{,}49 \qquad (XI)$$

Inasmuch as the instrument is utilized at a

constant mixture flow rate and the volumes of the cylinders for a given instrument remain constant, the constant $k_1$ will be known and thus the $HHV_s$ is renderded directly related to the cycle time of either the or the valveing system utilized.

A representative system operating control and measuring electronics implementation is shown in Figures 8 and 9. Figure 7 shows the sheet layout for Figures 8 and 9.

Thus, in Figure 8A, there is shown an analog board, in Figure 8B, a microprocessor board, in Figure 8C, a gas supply and flame safeguard system. Some details of the analog board of Figure 8A is shown in greater detail in Figure 9.

In Figure 8A there is shown the analog board P which includes an array of attached input thumb wheel switches as at 300 and indicates the connections with various other parts of the electronic system including the CMC 68/15 microprocessor control board of Figure 8B which is designated P′. The numbers and letters designated for the connection to P and P′ constitute the pin connections to the board P and P′. A series of pins beginning with P9 and ending with P38 on board P are adapted to line up and connect with pins P′43—P′67 of the CMC 68/15 and the composite of these lines comprise the address and data buses therebetween. Thumb wheel switches associated with the board P are provided to enter data associated with various peripheral functions associated with a particular model of the invention. Thus, such switches are provided to enter data concerning the time, date, colorie or heating value of a standard or a calibration gas to be used, high and low colorie limit (which are used in association with the metering system described below), etc. The function of the thumb wheel switches 300, including input and outlet are multiplexed in time in a well known manner.

As shown in Figure 8B, a CMC 68/15 interfaces with the display board P″ which is part of the display system 78 and through a printer interface 301 with a printer 80. A printer power supply is shown at 302. A stepper motor drive board 303 is provided to interface with the CMC 68/15 and to provide the control and modulation for this stepper motor 61 or 140. The CMC 68/15 includes conventional components contained in a conventional, commercially available electronic unit and is available from RCI/Data Corporation of Sattle Brook, New Jersey. The components include a microprocessor, a memory which includes a random acces memory portion (RAM), and an electrically programmed read only memory portion (EPROM), a parallel interface unit, timer, and display unit driver P″. These units are respectively connected by address buses and data buses in a conventional pre-assembled board. A serial interface and 1 MHz crystal oscillator clock are also provided.

The components utilized in one CMC 68/15 used in one embodiment were standard off-the-shel items and they are listed below as follows:

| | |
|---|---|
| Microprocessor 201 | MC6800 |
| Memory 202 | 2114 RAM |
| | 1Kx4 |
| | 2716 EPROM |
| | 2Kx8 |
| Parallel Interface 203 | MC 6821 |
| Timer 204 | MC6840 |
| Display Driver 25 | Litronics DL 1650 |
| Serial Interface 214 | 2651 Signetics |
| 4 MHz Crystal Oscillator | Knight |

Individual components available from various manufacturers such as Motorola, Texas Instruments, etc.

Figure 8C depicts the details of the flame safeguard and gas safety system which may be used with the present invention. In addition to the components described in relation to Figure 2 include solid state relays 310 and 311 and power supplies 312, 313, and 314. The customer safety shutdown may also be provided as shown by the dashed lines at 315.

In operation, when the system is started, air and gas flow are initiated through the particular system utilized and the burners ignited by the spark ignition in conventional fashion. The flame safeguard units 46 and 54 then indicate the presence of a flame through the signal lines P14 and P17. The signal is debounced as explained below in conjuction with Figure 9. The debounced signal causes the contacts 49 and 51 to close thus allowing the gas safety valve 56 (and the safety shutdown valve if used) to remain open.

The debouncing circuit is denoted by the dashed line labeled 316. The system basically includes an MC14490 debouncers which stabilizes the relay contacts of the flame safeguard device and also the inputs from the board P′ concerning the calibration command and output data command. Also shown on sheet a of Figure 9 are a threshold detector system indicated at 330 and circuitry associated with the one Hz interrupt clock which controls the program logic sequencing (discussed below) at 330.

The signal from the $ZrO_2$ sensor is compared to a predetermined threshold value in the threshold detector as by a comparator 321. At the point of stoichiometry, the output changes from a low signal to a high signal indicating that the point of stoichiometry has been passed going from air rich to fuel rich. As explained below,

when the system is tracking properly, the indication should alternate from air rich to fuel rich, etc., to show that the system is tracking on the point of stoichiometry.

The 1Hz interrupt clock system 330 includes a counter 331 and a comparator 332. The comparator 332 is utilized to determine when the counter 331 has reached 0 indicating that a full second has passed and it is time to activate the interrupt. The counter is then reset and begins timing for the next sequential interrupt. Counter 331 basically rides a conventional 60 Hz power input down until it equals a 1 Hz time clock.

The system indicated by the clock 330 in Figure 9B is basically an analog-to-digital converter system which converts analog data received at various parts of the system into a form usable by the central processing unit of the system. These include high and low temperature limits for the air and gas of the system along with actual values sensed and numbers pertaining to the calibration gas and high and low calorie limit set for the operation of the system as described in greater detail below. The system includes the constant current source at 331, typical analog multiplexes at 322 and 323, and a voltage-to-frequency converter 324 which are combined in a well known manner. The output of the VFO counter is sent to a timer in the CMC 68/15 where the signal is properly multiplexed and the periods measured.

It should also be mentioned in regard to Figure 10A that the sonalert circuit is shown at 345. The system is designed to sound an alarm when the calorie measurement is out of the limits that have been dialed in on the proper input thumb wheel switches. Circuit 345 provides the driver circuitry for this sonalert.

A representative software logic control schematic layout in accordance with the present invention will now be described in accordance with Figures 10—16. Figure 10 depicts a layout of the basic routines shown in greater detail in Figures 11—16. They include a main routine 400 with initialize subroutine 401, startup task 402, calibration task, 403, data logger task 404, display task 405 with associated time and date check 406, search task 407, display task 407, read temperature task 408. Other associated subroutine are shown at 409, 410 and 411. The main routine, of course, controls the interaction of the various tasks. A particular system described in relation to Figures 10—14 involves a system utilizing preferred rotary valve system as depicted in Figure 2.

Figure 11 depicts the logic diagram for the main routine in accordance with the preferred embodiment. As with all such systems, operation cannot begin until the entire system is activated or initialized as at 401. Upon power of the instrument, then, the first step in the software logic is to call the initialize subroutine which enters at 420 in Figure 11B. This happens as soon as the instrument is started and before the sampler vent burners are lit. The initilize subroutine then initializes the parallel interface at 421, the time module at 422, and the serial interface unit at 423. These modules are part of the 68/15 board and must be initialized and configured as predetermined in the system. Following this, the initialize subroutine exits at 424 and the main subroutine continues by transferring control from the initialize subroutine to the startup task 402. The startup task is found in Figure 12.

The logic flow enters at 430 and causes a startup message to be displayed on the display at 431 which tells observers the state of the instrument. The software then initializes the flame safeguard and sparking device to light the gas mixtures at both burners. During startup, the stepper motor is modulated by the frequency of the output of the timer model 422 which was initialized during the initialize subroutine. At startup, the frequency is varied to locate a mixture of air and fuel that will sustain a flame by increasing the values of frequency in accord with a lookup table in the microprocessor EPROM memory. Thus, after the gas valves allowed to be open as at 432 and the flame safeguard device started as at 433, the index 434 is initially at 0 or the first selected frequency in the table. If the flame safeguard system indicates the presence of a flame at 435, a five-minute delay is initiated at 436 to allow the sample gas burner to stabilize and the ceramic sensor to achieve its proper operating equilibrium temperature. At that point, the logic flow exits at 437.

If, on the other hand, presence of a flame is not indicated at 435, the stepper motor frequency is indexed to the next position at 438 and 439 such that the mixture is slightly enriched. A ten-second delay is implemented at 440 so that the mixture may stabilize at the burner. After this delay, the logic flow again is routed to 435 via 441 and the sequence repeated until flame is indicated to be present or 90 seconds passes at 442 in which case the flameout message 443 is displayed and the instrument is placed in a manual lockout state at 444 which requires the attention of service personnel.

Once the burner flame is stabilized and the startup logic flow exits at 437, the control returns again to the main routine which thereafter transfers control sequentially to the other tasks as controlled by the real time interrupt subroutine 409. The real time interrupt times out once every second as shown in Figure 9A and controls the timing of the running of each of the other tasks. That routine also keeps the date and the time current.

The first of these tasks is the call search task 407 which is shown in detail in Figure 13. Thus, when the real time interrupt indicates that the search task should be called, the logic flow enters on 450 and the search task is begun. It is

the search task that attempts to find the point of the stoichiometry mixture ratio and thereafter to track this ratio as closely as possible such that the sample burner mixture is maintained as close as possible to the stoichiometry point of the mixture of interest. As previously described, the $ZrO_2$ sensor provides a continuous feed-back signal dependent upon the partial pressure of oxygen in the sample combustion chamber. This feedback signal is continuously compared to a threshold or set point in the threshold detector. Thus, the first inquiry made by the search task at 451 is whether in fact a transition has taken place, i.e. whether the $ZrO_2$ feedback has crossed the threshold. If not, the status of the search timer is checked at 452 and if this is not timed out the logic flow exits at 453 until the next time the search task is called by the real time interrupt.

If no transition has taken place but the search timer has timed out, this indicates by the state of the threshold indicator that the mixture is either on the rich or lean side of the threshold or transition point and therefore a change in the ratio is indicated if it is to approach the stoichiometric or transition point. This is accomplished by first incrementing a no transition counter 454 which indicates that no transition has taken place for a given number of counts. If that given number is greater than 2 as at 455, the value of delta is checked. Delta is equal to a given amount related to the amount by which the frequency of the stepping motor is changed to speed up or slow down and thereby change the air-fuel ratio at the sample burner. The maximum delta is given a value of 128. Thus, if the no transition counter has a value greater than 2 and the value of delta at 456 is equal to 128 which is the maximum, the signal goes via 457 to 458 where a determination as to whether the mixture is air rich or not depending on the state of the threshold detector is made. If, however, delta does not equal the maximum of 128 at 456, delta is doubled at 459 and the increment no transition counter 454 is cleared at 460 and the logic flow proceeds to 458. If the no transition counter has not yet reached a value greater than 2, the logic flow also proceeds to 458. After the decision at 458, the stepper time is either increased or decreased by the amount delta 461 and 462, respectively, depending on whether the mixture is determined to be air rich or not. Of course, by slowing down the stepper time, the frequency of steps is decreased and the speed of rotation of the rotor is decreased thereby leaning the mixture. Thus, if the decision at 458 was that the mixture was fuel rich, the stepper time is increased by the amount delta thus slowing down the rotation by a given related amount. The stepper frequency is then compared with a maximum stepper time or slower rotor speed of 3300 microseconds. If the speed is greater than this at 464, it is reset to 3300 microseconds and the search task outputs a new frequency at 465 and 466 exiting at 467. Likewise, if the mixture is determined to be air rich, the stepper timer is compared at 468 which is slowest allowable speed and reset if necessary at 469 with the output again being modulated at 465 and 466.

In similar fashion, if the search task first indicates that in fact a transition has occurred, then the no transition counter 455 is cleared at 470 and the value of delta checked at 471. If delta is greater than 1, which is the minimum delta, then the inverse of the reaction at 459 occurs and the value of a delta is halved at 47 with the logic flow then proceeding to 458 where the same sequence just described occurs. The value of delta may vary from 1 to 128 and the purpose of doubling and halving delta is required to attempt to speed the search operation in an effort to bracket the transition point such that close tracking of the stoichiometric air-fuel ratio can be accomplished. As can be seen from the search task of Figure 13, if delta continually equals 1 and a transition takes place upon each search, a very low modulation about the stoichiometric point occurs and the system is truly tracking correctly, assuming that the mixture itself does not change. The search task is normally called about every two seconds which gives some delay time for the $ZrO_2$ sensor to stabilize after each change. After the search task logic flow exits at 467, control is returned to the main routine as interfaced by the real time interrupt. The rest of the main routine is concerned with a series of other tasks which are interfaced with the main routine by timers, which, upon timing out, enable the other tasks to gain control and be executed.

In this manner, if the calibration timer is equal to 0 when the logic flow is sequenced at 480, the calibration task 403 will be called. Other such timers include data logger timer 481, display timer 482, read temperature timer 483, and two panel initiated buttons including a calibrate button and a print button 484 and 485, respectively.

Assuming that the calibration timer is timed out, or that the push button 484 has been pushed, the main routine relinquishes control to the calibration task of Figure 14. In this task, the logic flow enters as at 500 and selects the calibration gas at 501. This causes the three-way inlet valve to switch from sample gas or gas of interest to calibration gas as the fuel gas. After a 30-second delay to allow the burner to stabilize at 502, the system is checked as to whether it is tracking at 503 prior to search routine 407. If not, this is checked continually on a one-second delay as at 504 until tracking occurs at which time the read temperature task 408 (discussed below) is called. Based on the output of the read temperature task 408, the constant $K_1$ utilized in the BTU calculation as described is recalculated utilizing any necessary temperature correction as at 505 and the

inlet valve is again switched back to the sample or unknown gas of interest as at 506 and the logic flow exits at 507 returning control back to the main task. The method of correction for temperature is discussed below in relation to the read temperature task in Figure 16. The calibration timer is reloaded at 487 and calibration will not occur automatically until the calibration timer again times out. The calibration timer is normally set for a period of up to 12 hours such that if calibration is successful, it occurs only rarely in the operation of a device.

When the data logger timer 431 times out, the data logger task 404 is called to cause the data at 487 to be printed and thereafter the data logger timer is reloaded at 488. This routine allows certain data to be periodically printed to monitor the operation of the system.

In a like manner, the display task 405 is called and, as seen in Figure 15, logic flow enters at 510 and asks the status of the time and date thumb wheels as at 511. Thus, the display can update and time the date as at 513 and 514, calculate the calories or heating value of the fuel from the floating point mathematics as at 515, and display the results as at 516. Returning to the main routine, again the display timer is reloaded as at 489. In a like manner, the main task executes the read temperature task 408. This task is shown in detail in Figure 16. The logic flow enters at 520, and the input from the calibration resistor which is the standard against which the temperature sensor is compared is called at 521. The voltage-to-frequency routine is called at 522 which outputs a frequency signal based on the calibration resistor. In a similar manner, at 523—526, frequency signals are generated for the air temperature sensor and the mixture temperature sensor and from these at 527 and 528 the resistance of the air sensor and mixture sensor can be calculated and the air and mixture temperature is derived at 529 and 530.

After completing one cycle of the main routine, the routine is returned to being timing through the tasks again as at 490. Thus, the routine begins all over again and continues to continually go through the various routines as determined by the real time interrupt and the various times associated with each routine.

The mechanical components of the system of Figures 2 may be any conventional components which meet the requirements of the system. These include the pressure regulators, temperature sensors, flame safeguards, safety and ignition systems, and burners. These are all conventoinal items available from a variety of manufactures.

In the embodiment of Figure 2, the stepping motor used in one successful embodiment was a 6000 series SLO-SYN stepping motor available from the Superior Electric Company of Bristol, Connecticut. These motors have a 1.8° step angle or a total of 200 steps per revolution of the shaft. Thus, each control input can step the motor as little as 1.8°. The flexible coupling 105 used in one successful embodiment in conjunction with the stepping motor was an MCA 100 HELI-CAL flexible coupling manufactured by the Helical Products Company, inc. of Santa Maria, California.

It can readily be seen from the above description that the several proportioning systems which have been described in accordance with the heat content measuring system of the present invention may have many applications elsewhere. These proportioning systems have characteristics that make them quite useful for the accurate mixing of all types of gases and even liquids in association with other systems.

As can be seen from the above description, the heat content measuring system of the present invention including the several alternate proportioning systems provides an effective, simple, and accurate system for determining the heat content of gaseous hydrocarbon fuels. Results utilizing the invention indicate that very high accuracies can be obtained readily.

## Claims

1. An apparatus for determining the volumetric heat content of a gaseous fuel, said apparatus comprising:

(a) a mixing device (60) for establishing a mixture of air and the gaseous fuel;

(b) means (44) for combusting the fuel in said mixture;

(c) a sensor (48) for sensing a physical quantity related to the result of the oxidation involved in the combustion;

(d) means (70) for adjusting the air to fuel volumetric ratio in said mixture in response to the output of said sensor until said sensor indicates that the degree of oxidation corresponds to substantially stoichiometric combustion;

(e) means (38, 70) for measuring the air to fuel volumetric ratio when achieving substantially stoichiometric combustion;

(f) means (70) for determining the volumetric heat content of said fuel from said volumetric air to fuel ratio measured in the state of substantially stoichiometric combustion by using a known relationship between the stoichiometric values for the different constituents of said fuel and the corresponding volumetric heat contents; characterized in that,

(g) said sensor (48) is a solid state electrochemical sensor for sensing the presence of combustibles or oxygen and provides a rapidly changing output signal when substantially stoichiometric combustion is reached;

(h) said mixing device is a rotary proportioning system (60) comprising at least
(h1) a first inlet (62, 101) adapted to be connected to a source of the fuel;
(h2) a second inlet (63, 101) adapted to be connected to a source of the air;
(h3) a first outlet (64, 102) for discharging the mixture of interest;
(h4) a rotatable member (100) which is provided with a plurality of fixed hollow volumes (122) each having an open access at both sides of the rotatable member (100);
(h5) first connecting means (118, 119) provided stationarily at the one side of the rotatable member (100) for connecting said inlets (62, 63) to the accesses on one side of the rotatable member;
(h6) second connecting means (120, 121) for connecting said first outlet (64) to the accesses on the other side of the rotatable member (100);
(h7) a motor (61) for rotating said rotatable member (100).

2. Apparatus according to claim 1, characterized in that for discharging an exhaust mixture a second outlet (65, 102) is provided stationarily at the other side of the rotatable member (101) and the first and second connecting means (118—121) are arranged such that upon rotation of said rotatable member (100) each of said hollow volumes is sequentially connected between said first inlet (62) and said second outlet (65) and said second inlet (63) and said first outlet (64).

3. Apparatus according to claim 1 or 2, characterized in that the second connecting means (120, 121) are provided at the opposite side of the rotatable member (100).

4. Apparatus according to one of claims 1 to 3, characterized in that

(i) said rotatable member (100) comprises a cylindrical rotor;
(j) said hollow volumes (122) are formed by cylindrical holes extending in axial direction through said rotor (100) and distributed around the circumference of said rotor;
(k) said first connecting means comprises a first partially annular inlet groove (119) provided in a first stationary distribution plate (101) on the side facing the inlet accesses of said hollow volumes (122) as well as a second partially annular inlet groove (118) provided in said first distribution plate (101) on the same side but spaced in circumferential direction from said first inlet groove;
(l) said second connecting means comprises a first partially annular outlet groove (121) provided in a second stationary distribution plate (102) on the side facing the outlet accesses of said hollow volumes (122) as well as a second partially annular outlet groove (120) provided in said second distribution plate (102) on the same side but spaced in circumferential direction from said first outlet groove (121).

5. Apparatus according to claim 4, characterized in that said first (119) and second (118) inlet grooves are of different length, and said first (121) and second (120) outlet grooves are of different length.

6. Apparatus according to claim 1 or 2, characterized in that

(m) said rotatable member (144, 145) comprises a cylindrical rotor (144) provided with dual inner and outer series of spaced hollow volumes (164) extending in axial direction and distributed around the circumference of said cylinder and forming a symmetrical pattern such that each inner hollow volume is paired with a corresponding outer hollow volume;
(n) both the first and second inlets (160, 162) and outlets (161, 163) as well as the first and second connecting means (147—150) are stationarily provided at the same side of the rotatable member (144, 145),
(o) a cylindrical base plate (145) attached to said rotor (144) has radially extending rabbets (165) provided in its surface which engages said rotor, said rabbets connecting the base portions of each pair of the inner and outer hollow volumes therewith forming an U-shaped conduit (164, 165) in said rotatable member (144, 145);
(p) inner (148, 150) and outer (147, 149) pairs of parallel fixed partially annular grooves forming the connecting means (147, 150) are juxtaposed to and aligned with said inner and outer series of hollow volumes (164), each of said pairs of grooves comprising a shorter (149, 150) and a longer groove (147, 148) spaced apart and, wherein the two shorter and the two longer grooves are in radial alignment;
(q) one (149) of said shorter grooves (149, 150) is connected to the fuel inlet (162) and the other (150) of said shorter grooves connects to an exhaust outlet (163);
(r) one of said longer grooves (147, 148) is connected to the air inlet (160) and the other of said longer grooves provides an outlet (161) for said mixture of interest.

7. Apparatus according to claim 4, 5 or 6, characterized in that inlet (160, 162) and said outlet connecting means (161, 163) comprise openings (127—130, 155—159) connecting said grooves (118—121; 147—150) and wherein the corresponding openings connecting said inlet grooves and said outlet grooves are located at opposite extremes of said grooves such that the contents of the grooves also tends to flow along said grooves in transversing the system.

8. Apparatus according to claim 7 characterized in that the disposition of said inlet and

outlet openings (127—130; 155—159) accessing said grooves (118—121; 147 to 150) relative to the direction of rotation of said rotatable member (100, 144) is such that the fuel tends to flow along the inlet in a direction opposite to the rotation of the rotatable member and the air flow tends to be in the same direction as the rotation of the rotatable member.

9. Apparatus according to one of claims 1 to 8, characterized in that the motor (61, 140) is a stepping motor.

10. Apparatus according to one of claims 1 to 9, characterized by a controller (70) for controlling and varying the speed of said motor (61, 140).

11. Apparatus according to one of claims 1 to 10, characterized by said sensor (48) comprising an electrochemical cell, the output signal of which undergoes a step change as the amount of oxygen sensed approaches zero.

12. An apparatus according to claim 11, characterized in that the electrochemical cell is made substantially of $ZrO_2$ stabilized by a compound substantially consisting of one or more of the following, CaO, MgO and $Y_2O_3$.

13. An apparatus according to one of claims 1 to 12, characterized by regulators (32, 42) for the flow of fuel and air entering the system.

14. Apparatus according to one of claims 1 to 13, characterized by calibration means (35—37; 206—208) for calibrating said known relationship between the stoichiometric values for the different constituents of said fuel and the corresponding volumetric heat contents.

**Patentansprüche**

1. Vorrichtung zur Bestimmung des volumetrischen Wärmeinhalts eines gasförmigen Brennstoffs mit:

(a) einer Mischvorrichtung (60) zum Herstellen einer Mischung aus Luft und dem gasförmigen Brennstoff;
(b) einer Vorrichtung (44) zum Verbrennen des in der Mischung enthaltenen Brennstoffs;
(c) einem Fühler (48) zum Feststellen einer physikalischen Größe, welche zum Ergebnis der bei der Verbrennung auftretenden Oxidation in Beziehung steht;
(d) einer Einrichtung (70), um das volumetrische Luft/Brennstoff-Verhältnis in der Mischung in Abhängigkeit vom Ausgangssignal des Fühlers solange einzustellen, bis der Fühler anzeigt, daß der Grad der Oxidation einer praktisch stöchiometrischen Verbrennung entspricht;
(e) einer Meßeinrichtung (38, 70) für das bei praktisch stöchiometrischer Verbrennung gegebene Luft/Brennstoff-Verhältnis;
(f) einer Einrichtung (70), welche aus dem volumetrischen Luft/Brennstoff-Verhältnis im Zustand praktisch stöchiometrischer Verbrennung den volumetrischen Wärmeinhalt des Brennstoffs bestimmt und hierzu eine bekannte Beziehung zwischen den stöchiometrischen Werten der verschiedenen Bestandteile des Brennstoffs und den entsprechenden volumetrischen Wärmeinhalten benutzt, dadurch gekennzeichnet, daß
(g) der Fühler (48) ein elektrochemischer Festkörperfühler ist, der auf die Anwesenheit von brennbaren Bestandteilen oder Sauerstoff anspricht und beim Erreichen praktisch stöchiometrischer Verbrennung eine steile Änderung seines Ausgangssignals liefert;
(h) die Mischvorrichtung aus einer rotierenden Zuteileinrichtung (60) besteht mit wenigstens
(h1) einem ersten Einlaß (62, 101) zum Anschluß an eine Brennstoffquelle;
(h2) einem zweiten Einlaß (63, 101) zum Anschluß an eine Luftquelle;
(h3) einem ersten Auslaß (64, 102), welcher die gesuchte Mischung abgibt;
(h4) einem drehbaren Körper (100) mit einer Vielzahl vorgegebener Hohlräume (122), von denen jeder an beiden Seiten des drehbaren Körpers (100) einen offenen Zugang aufweist;
(h5) einer ersten ortsfesten Anschlußvorrichtung (118, 119) auf der einen Seite des drehbaren Körpers (100) welche die Einlässe (62, 63) mit den Zugängen auf der einen Seite des drehbaren Körpers verbindet;
(h6) einer zweiten Anschlußvorrichtung (120, 121), welche den ersten Auslaß (64) mit dem Zugang auf der anderen Seite des drehbaren Körpers (100) verbindet;
(h7) einem Motor (61) zum Drehen des drehbaren Körpers (100)

2. Vorrichtung nach Anspruch 1, dadurch gekennzeichnet, daß zum Ausstroßen einer Abgasmischung ein zweiter Auslaß (65, 102) ortsfest an der anderen Seite des drehbaren Körpers (100) vorgesehen und die ersten und zweiten Anschlüsse (118—121) derart angeordnet sind, daß bei Drehung des drehbaren Körpers (100) jeder der Hohlräume nacheinander zwischen den ersten Einlaß (62) und den zweiten Auslaß (65) sowie zwischen den zweiten Einlaß (63) und den ersten Auslaß (64) eingeschaltet wird.

3. Vorrichtung nach Anspruch 1 oder 2, dadurch gekennzeichnet, daß die zweiten Anschlüsse (120, 121) auf der gegenüberliegenden Seite des drehbaren Körpers (100) angeordnet sind.

4. Vorrichtung nach einem der Ansprüche 1 bis 3, dadurch gekennzeichnet, daß

(i) der drehbare Körper (100) einen zylindrischen Rotor aufweist;
(j) die Hohlräume (122) durch zylindrische, sich in Achsrichtung durch den Rotor (100) hindurch erstreckende Löcher gebildet und über den Umfang des Rotors verteilt angeordnet sind;

(k) die ersten Anschlüsse eine erste teilringförmige Einlaßnut (119) umfassen, welche in einer ersten ortsfesten Verteilerplatte (101) auf der den Einlaßzugängen der Hohlräme (122) zugewandten Seite vorgesehen ist, sowie eine zweite teilringförmige Einlaßnut (118) enthalten, die in der ersten Verteilerplatte (101) auf der gleichen Seite aber in Umfangsrichtung gegenüber der ersten Einlaßnut versetzt angeordnet ist;

(l) die zweiten Anschlüsse eine erste teilringförmige Auslaßnut (121) umfassen, welche in einer zweiten ortsfesten Verteilerplatte (102) auf der den Auslaßzugängen der Hohlräume (122) zugewandten Seite vorgesehen ist, sowie eine zweite teilringförmige Auslaßnut (120) enthält, die in der zweiten Verteilerplatte (102) auf der gleichen Seite aber in Umfangsrichtung gegenüber der ersten Auslaßnut (121) versetzt angeordnet ist.

5. Vorrichtung nach Anspruch 4, dadurch gekennzeichnet, daß sowohl die ersten (119) und zweiten (118) Einlaßnuten als auch die ersten (121) und zweiten (120) Auslaßnuten jeweils unterschiedlich lang sind.

6. Vorrichtung nach Anspruch 1 oder 2, dadurch gekennzeichnet, daß

(m) der drehbare Körper (144, 145) einen zylindrischen Rotor (144) aufweist, der mit paarweisen inneren und äußeren Gruppen von sich im Abstand voneinander in Achsrichtung erstreckenden und über den Umfang des Zylinders verteilt angeordneten Hohlräumen (164) versehen ist, die ein symmetrisches Muster dergestalt bilden, daß jeder innere Hohlraum mit einem entsprechenden äußeren Hohlraum ein Paar bildet;

(n) sowohl die ersten und zweiten Einlässe (160, 162) und Auslässe (161, 163) als auch die ersten und zweiten Anschlüsse (147—150) ortsfest auf der gleichen Seite des drehbaren Körpers (144, 145) angeordnet sind;

(o) eine am Rotor (144) befestigte zylindrische Grundplatte (145) sich radial erstreckende Verteifungen (165) in der dem Rotor zugewandten Oberfläche aufweist, welche jeweils die Endteile jedes aus einem inneren und einem äußeren Hohlraum bestehenden Paars verbinden und somit einen U-förmigen Kanal (164, 165) im drehbaren Körper (144, 145) bilden;

(p) innere (148, 150) und äußere (147, 149) Paare ortsfester, paralleler teilringförmiger Nuten als Anschlüsse (147—150) gegenüber und fluchtend mit den inneren und äußeren Hohlräumen (164) angeordnet sind, wobei jedes Nutpaar im Abstand voneinander eine kürzere (149, 150) und eine längere (147, 148) Nut aufweist und die beiden kürzeren und die beiden längeren Nuten radial fluchten;

(q) eine (149) der kürzeren Nuten (149, 150) an den Brennstoffeinlaß (162) und die andere (150) an den Abgasauslaß (163) angeschlossen ist;

(r) eine der längeren Nuten (147, 148) mit dem Lufteinlaß und die andere mit dem Auslaß (161) für die gesuchte Mischung in Verbindung steht.

7. Vorrichtung nach Anspruch 4, 5 oder 6, dadurch gekennzeichnet, daß die Einlaßanschlüsse (160, 162) und die Nuten (118—121; 147—150) verbindende Öffnungen (127—130; 155—159) aufweisen, wobei die einander zugeordneten, die Einlaßund die Auslaßnuten verbindenden Öffnungen an gegenüberliegenden Enden der Nuten derart vorgesehen sind, daß der Inhalt der Nuten beim Durchlaufen der Vorrichtung längs der Nuten zu strömen sucht.

8. Vorrichtung nach Anspruch 7, dadurch gekennzeichnet, daß die Anordnung der Einlaß- und Auslaßöffnungen (127—130; 155—159) als Zugang zu den Nuten (118—121; 147—150) in Bezug auf die Drehrichtung des drehbaren Körpers (100, 144) derart getroffen ist, daß der Brennstoff im Einlaß der Drehrichtung des drehbaren Körpers entgegenzuströmen sucht, während die Luft in Drehrichtung zu strömen sucht.

9. Vorrichtung nach einem der Ansprüche 1 bis 8, dadurch gekennzeichnet, daß der Motor (61, 140) ein Schrittmotor ist.

10. Vorrichtung nach einem der Ansprüche 1 bis 9, gekennzeichnet durch einen Regler (70) zum Steuern und Verändern der Drehzahl des Motors (61, 140).

11. Vorrichtung nach einem der Ansprüche 1 bis 10, dadurch gekennzeichnet, daß der Fühler (48) eine elektrochemische Zelle enthält, deren Ausgangssignal eine stufenförmige Änderung Zeigt, sobald die gemessene Sauerstoffmenge nach Null geht.

12. Vorrichtung nach Anspruch 11, dadurch gekennzeichnet, daß die elektrochemische Zelle im wesentlichen aus $ZrO_2$ besteht, welches durch eine im wesentlichen aus einem oder mehreren der folgenden Stoffe CaO, MgO und $Y_2O_3$ bestehende Verbindung stabilisiert ist.

13. Vorrichtung nach einem der Ansprüche 1 bis 12, gekennzeichnet durch Regler (32, 42) für die Brennstoff- und Luftzufuhr zur Vorrichtung.

14. Vorrichtung nach einem der Ansprüche 1 bis 13, gekennzeichnet durch eine Eichvorrichtung (35—37; 206—208) zum Eingeben der bekannten Beziehung zwischen den stöchiometrischen Werten für die einzelnen Bestandteile des Brennstoffs und den zugehörigen volumetrischen Wärmeinhalten.

**Revendications**

1. Appareil pour déterminer le contenu calorifique volumétrique d'un combustible gazeux, comprenant:

(a) un dispositif mélangeur (60) pour établir un mélange d'air et de combustible gazeux;

(b) un moyen (44) pour réaliser la combustion du combustible dans le mélange;

(c) uncapteur (48) pour capter une quantité physique relative au résultat de l'oxydation impliquée dans la combustion;

(d) un moyen (70) pour régler le taux volumétrique d'air par rapport au combustible dans le mélange en réponse au signal de sortie du capteur jusqu'à ce que ce capteur indique que le degré d'oxydation correspond à une combustion essentiellement stoechiométrique;

(e) un moyen (38, 70) pour mesurer le taux volumétrique d'air par rapport au combustible quand une combustion essentiellement stoechiométrique est obtenue;

(f) un moyen (7) pour déterminer le contenu calorifique volmétrique du combustible à partir du taux volumétrique d'air par rapport au combustible mesuré dans l'état de combustion essentiellement stoechiométrique en utilisant une relation connue entre les valeurs stoechiométriques pour les différents éléments constitutifs du combustible et les contenus calorifiques volumétriques correspondants; caractérisé en ce que:

(g) ledit capteur (48) est un capteur électrochimique à l'état solide pour capter la présence de combustibles ou d'oxygène et qui fournit un signal de sortie variant rapidement quand une combustion essentiellement stoechiométrique est obtenu;

(h) ledit dispositif mélangeur est un système de classement rotatif (60) comprenant au moins

(h1) une première entrée (62, 101) agencée pour être raccordée à une source du combustible;

(h2) une seconde entrée (63, 101) agencée pour être raccordée à une source d'air;

(h3) une première sortie (64, 102) pour évacuer le mélange intéressant;

(h4) un élément tournant (100) qui est pourvu d'un ensemble de volumes creux fixes (122) comportant chacun une ouverture d'accès des deux côtés de l'élément tournant (100);

(h5) des premiers moyens de connexion (118, 119) prévus d'une manière stationnaire d'un des côtés de l'élément tournant (100) pour relier lesdites entrées (62, 63) aux ouvertures d'accès d'un côté de l'élément tournant;

(h6) des seconds moyens de connexion (120, 121) pour relier la première sortie (64) aux ouvertures d'accès de l'autre côté de l'élément tournant (100);

(h7) un moteur (61) pour faire tourner l'élément tournant (100).

2. Appareil selon la revendication 1, caractérisé en ce qu'une seconde sortie (65, 102) est prévue d'une manière stationnaire de l'autre côté de l'élément tournant (101) pour évacuer un mélange d'échappement et en ce que les premiers et seconds moyens de connexion (118—121) sont disposés de sorte qu'à la rotation de l'élément tournant (100) chacun des volumes creux est relié séquentiellement entre la première entrée (62) et la seconde sortie (65) et la seconde entrée (63) et la première sortie (64).

3. Appareil selon l'une quelconque des revendications 1 et 2, caractérisé en ce que les seconde moyens de connexion (120, 121) sont prévus du côté opposé de l'élément tournant (100).

4. Appareil selon quelconque des revendications 1 à 3, caractérise en ce que;

(i) l'élément tournant (100) constitue un rotor cylindrique;

(j) les volumes creux (122) sont formés par des trous cylindriques s'étendant dans la direction axiale du rotor (100) et sont répartis autour de la périphérie du rotor;

(k) les premiers moyens de connexion comprennent une première rainure d'entrée partiellement annulaire (119) prévue dans une première plaque de répartition fixe (101) du côté situé en face des ouvertures d'accès d'entrée des volumes creux (122) ainsi qu'une seconde rainure de'entrée partiellement annulaire (118) prévue dans la première plaque de répartition (101) du même côté mais espacée dans la direction périphérique de la première rainure d'entrée;

(l) les seconds moyens de connexion comprennent une première rainure de sortie partiellement annulaire (121) prévue dans une seconde plaque de répartition fixe (102) du côté situé en face des ouvertures d'accès de sortie des volumes creux (122) ainsi qu'une seconde rainure de sortie partiellement annulaire (120) prévue dans la seconde plaque de répartition (102) du même côté mais espacée dans la direction périphérique de la première rainure de sortie (121).

5. Appareil selon la revendication 4, caractérisé en ce que les premières (119) et secondes (118) rainures d'entrée sont de longueurs différentes, et en ce que les premières (121) et secondes (120) rainures de sortie sont de longueurs différentes.

6. Appareil selon l'une quelconque des revendications 1 et 2, caractérisé en ce que:

(m) l'élément tournant (144, 145) constitue un rotor cylindrique (144) pourvu d'une double serie intérieure et extérieure de volumes creux (164) espacés s'étendant dans une direction axiale et répartis autour de la périphérie du cylindre et ayant une configuration symétrique telle que chaque volume creux intérieur est apparié avec un volume creux extérieur correspondant;

(n) les première et seconde entrées (160, 162) et les première et seconde sorties (161, 163) ainsi que les premiers et seconde moyens de connexion (147—150) sont prévus d'une manière stationnaire du même côté de l'élément tournant (144, 145);

(o) une plaque de base cylindrique (145) fixée au rotor (144) comporte des évidements (165) s'étendent radialement prévus à sa surface qui est mise en contact avec le rotor, lesdits évidements reliant les parties de base de chaque paire de volumes creux intérieur et extérieur en formant avec celles-ci un conduit en forme de U (164, 165) dans l'élément tournant (144, 145);

(p) des paires intérieures (148, 150) et extérieures (147, 149) de rainures partiellement annulaires fixes et parallèles constituant les moyens de connexion (147—150) sont juxtaposées à et alignées avec les séries intérieure et extérieure de volumes creux (164), chacune des paires de rainures comprenant une rainure plus courte (149, 150) et une rainure plus longue (147, 148) espacées l'une de l'autre, les deux rainures plus courtes et les deux rainures plus longues étant alignées radialement;

(q) une (149) des rainures plus courtes (149, 150) est reliée à l'entrée de combustible (162) et l'autre (150) des rainures plus courtes est reliée à une sortie d'échappement (163);

(r) une des rainures plus longues (147, 148) est reliée à l'entrée d'air (160) et l'autre des rainures plus longues fournit une sortie (161) pour ledit mélange intéressant.

7. Appareil selon l'une quelconque des revendications 4 à 6, caractérisé en ce que les moyens de connexion d'entrée (160, 162) et de sortie (161, 163) comprennent des ouvertures (127—130, 155—159) reliant lesdites rainures (118—121; 147—150) et en ce que les ouvertures correspondantes reliant les rainures d'entrée et les rainures de sortie sont placées à des extrémités opposées des rainures de sorte que le contenu des rainures tend également à passer le long des rainures en traversant le système.

8. Appareil selon le revendication 7, caractérisé en ce que la disposition des ouvertures d'entrée et de sortie (127—130; 155—159) donnant accès aux rainures (118—121; 147—150) par rapport au sens de rotation de l'élément tournant (100, 144) est telle que le combustible tend à passer le long de l'entrée dans un sens inverse au sens de rotation de l'élément tournant et que l'air tend à passer dans le même sens que le sens de rotation de l'élément tournant.

9. Appareil selon l'une quelconque des revendications 1 à 8, caracterisé en ce que le moteur (61, 140) est un moteur pas-à-pas.

10. Appareil selon l'une quelconque des revendications 1 à 9, caractérisé en ce qu'il comprend un dispositif de commande (70) pour commander et faire varier la vitesse du moteur (61, 140).

11. Appareil selon l'une quelconque des revendications 1 à 10, caractérisé en ce que le capteur (48) constitue une cellule électrochimique dont le signal de sortie subit une variation échelonnée quand la quantité d'oxygène captée s'approche de zéro.

12. Appareil selon la revendication 11, caractérisé en ce que la cellule électrochimique est constituée essentiellement de $ZrO_2$ stabilisé par un composé constitué essentiellement d'un ou de plusieurs des éléments constitutifs suivants: CaO, MgO et $Y_2O_3$.

13. Appareil selon l'une quelconque des revendications 1 à 12, caractérisé en ce qu'il comprend des régulateurs (32, 42) de dèbit de combustible et d'air pénétrant dans le système.

14. Appareil selon l'une quelconque des revendications 1 à 13, caractérisé en ce qu'il comprend un moyen d'étalonnage (35—37; 206—208) pour étalonner ladite relation connue entre les valeurs stoechiométriques pour les différents élément constitutifs du combustible et les contenus calorifiques volumétriques correspondants.

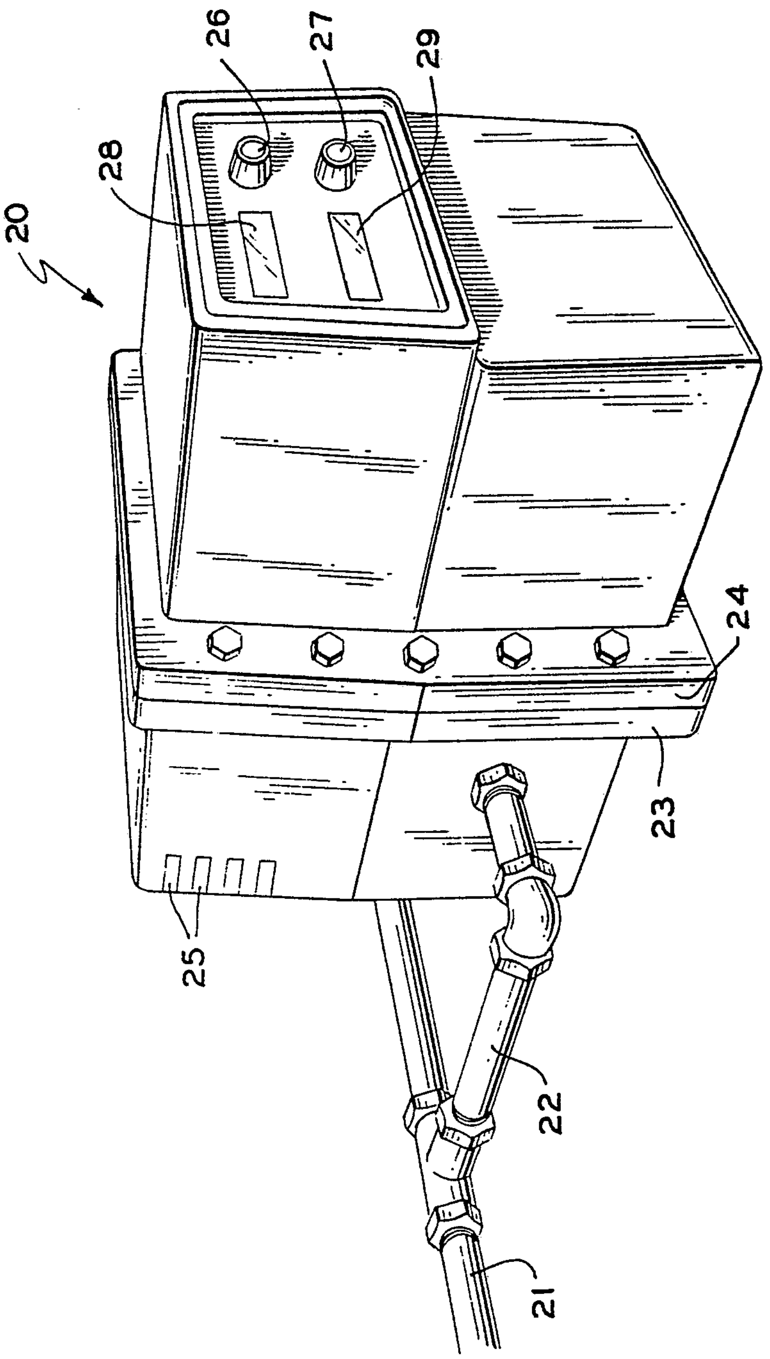


FIG. 1

*FIG. 2*

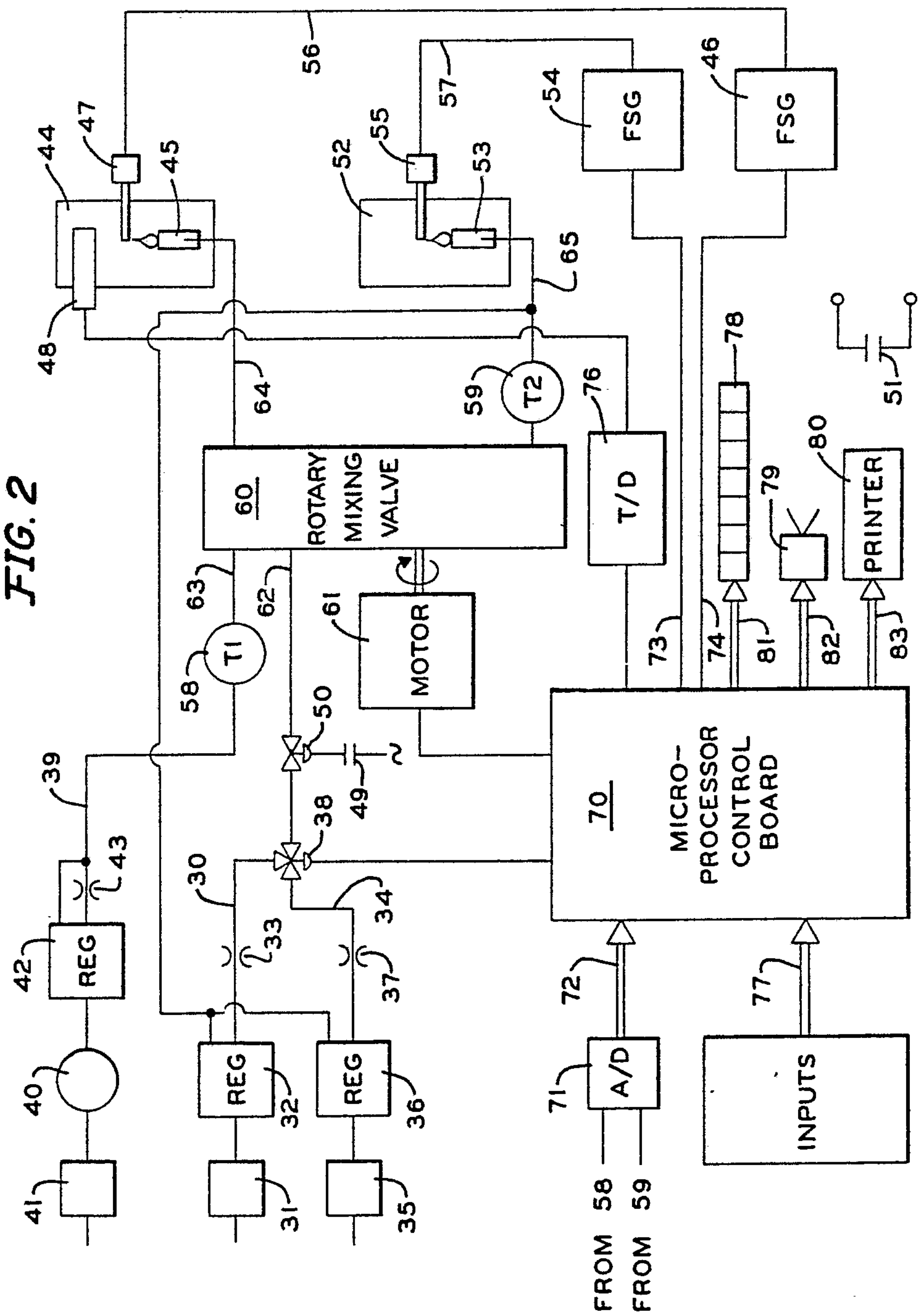
FSG
FSG
60
ROTARY
MIXING
VALVE
MOTOR
T/D
T1
T2
PRINTER
70
MICRO-
PROCESSOR
CONTROL
BOARD
REG
REG
REG
A/D
INPUTS
FROM 58
FROM 59

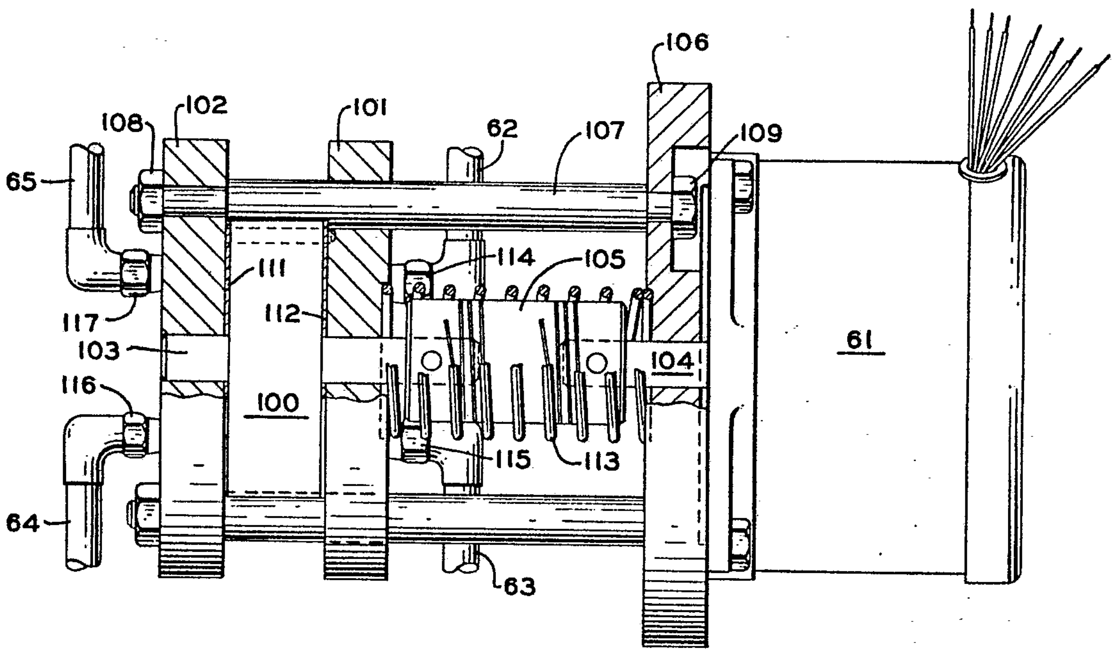

106
108
102
101
62
107
109
65
114
105
111
117
112
61
103
104
116
100
115
113
64
63


FIG. 3

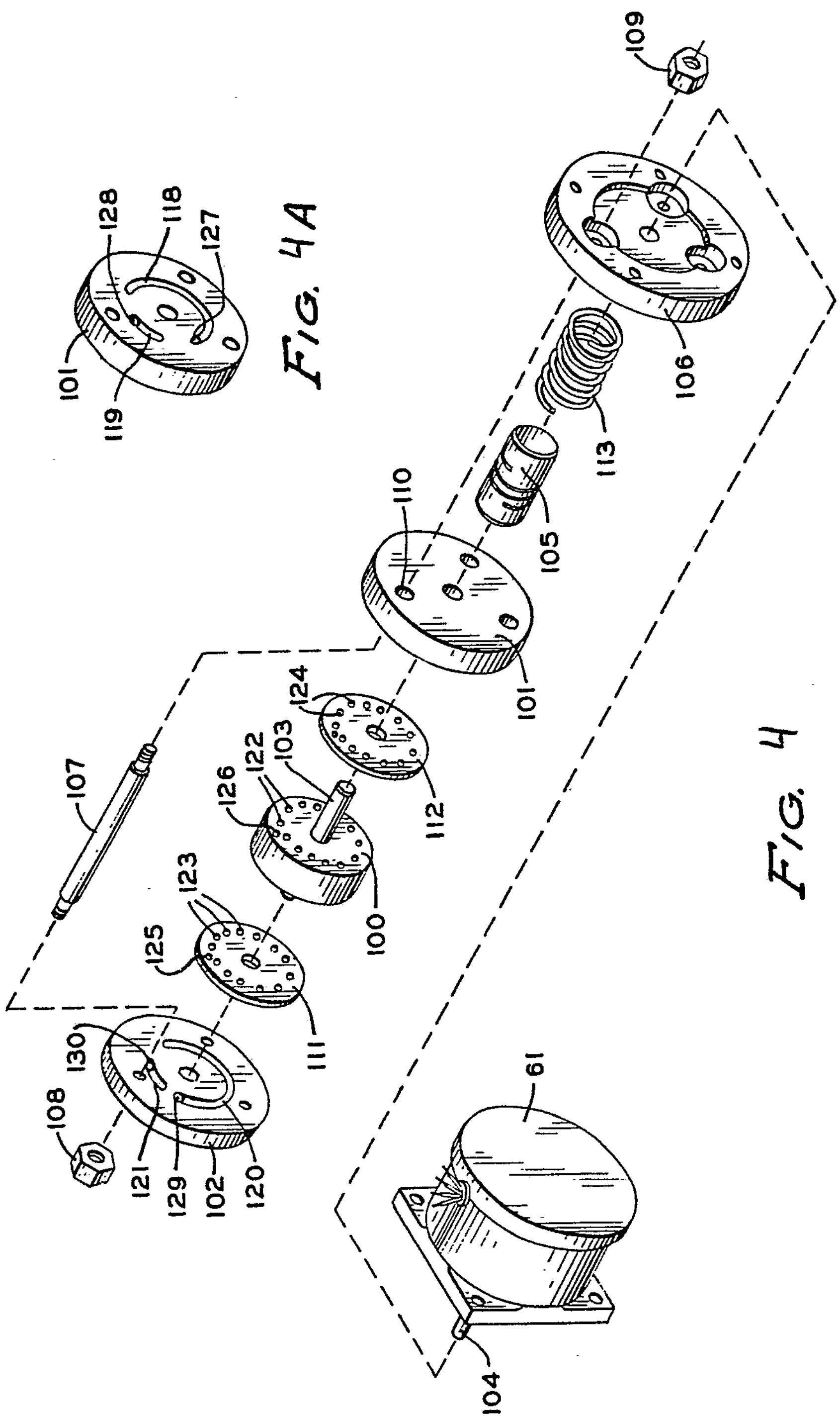
101
119
128
118
127
109
110
105
113
106
107
124
122
103
126
112
100
123
125
111
130
108
121
129
102
120
61
104

FIG. 4A

FIG. 4

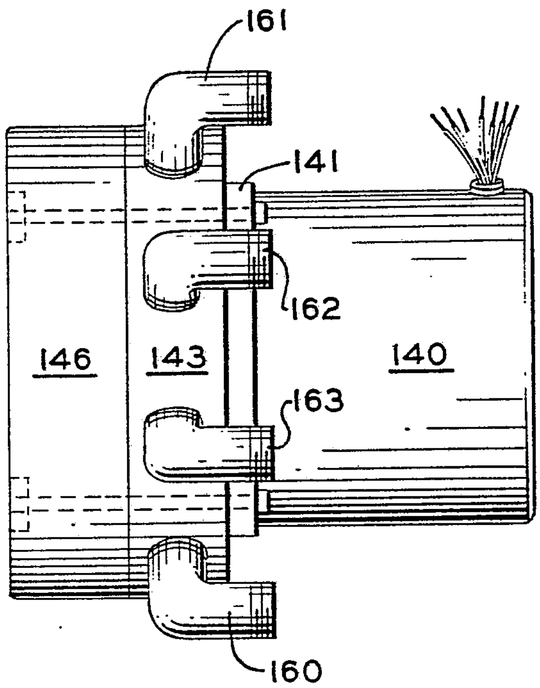


FIG. 5

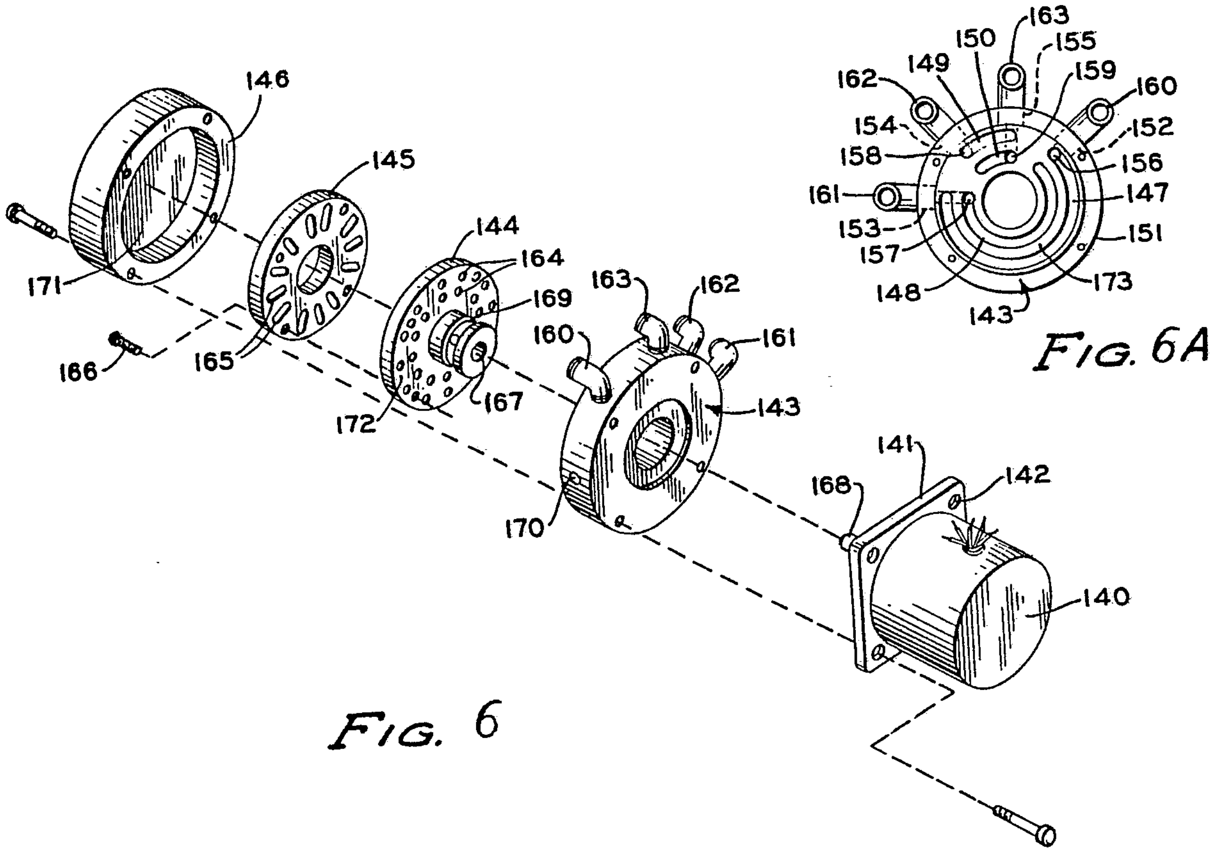
146
145
144
164
169
171
166
165
172
167
163
162
160
161
143
141
142
168
170
140

FIG. 6

163
150
155
149
159
162
160
154
152
158
156
161
147
153
151
157
173
148
143

FIG. 6A

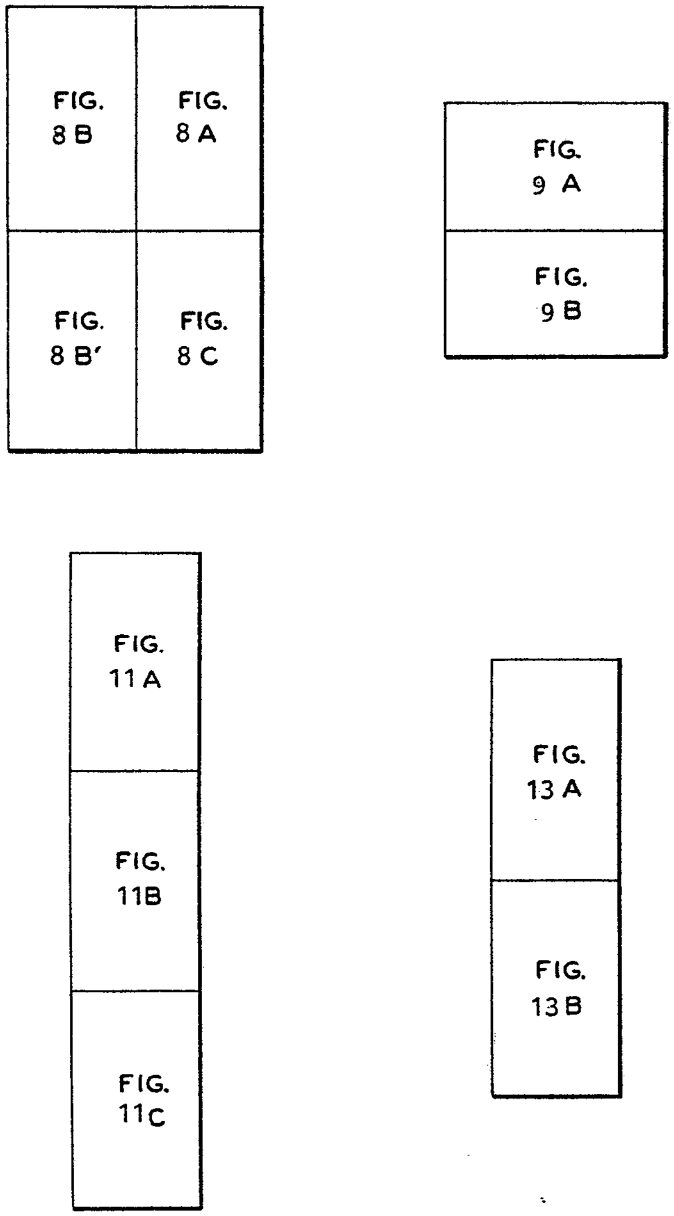
FIG. 8 B
FIG. 8 A
FIG. 8 B'
FIG. 8 C
FIG. 9 A
FIG. 9 B
FIG. 11 A
FIG. 11B
FIG. 11C
FIG. 13 A
FIG. 13 B

FIG. 7

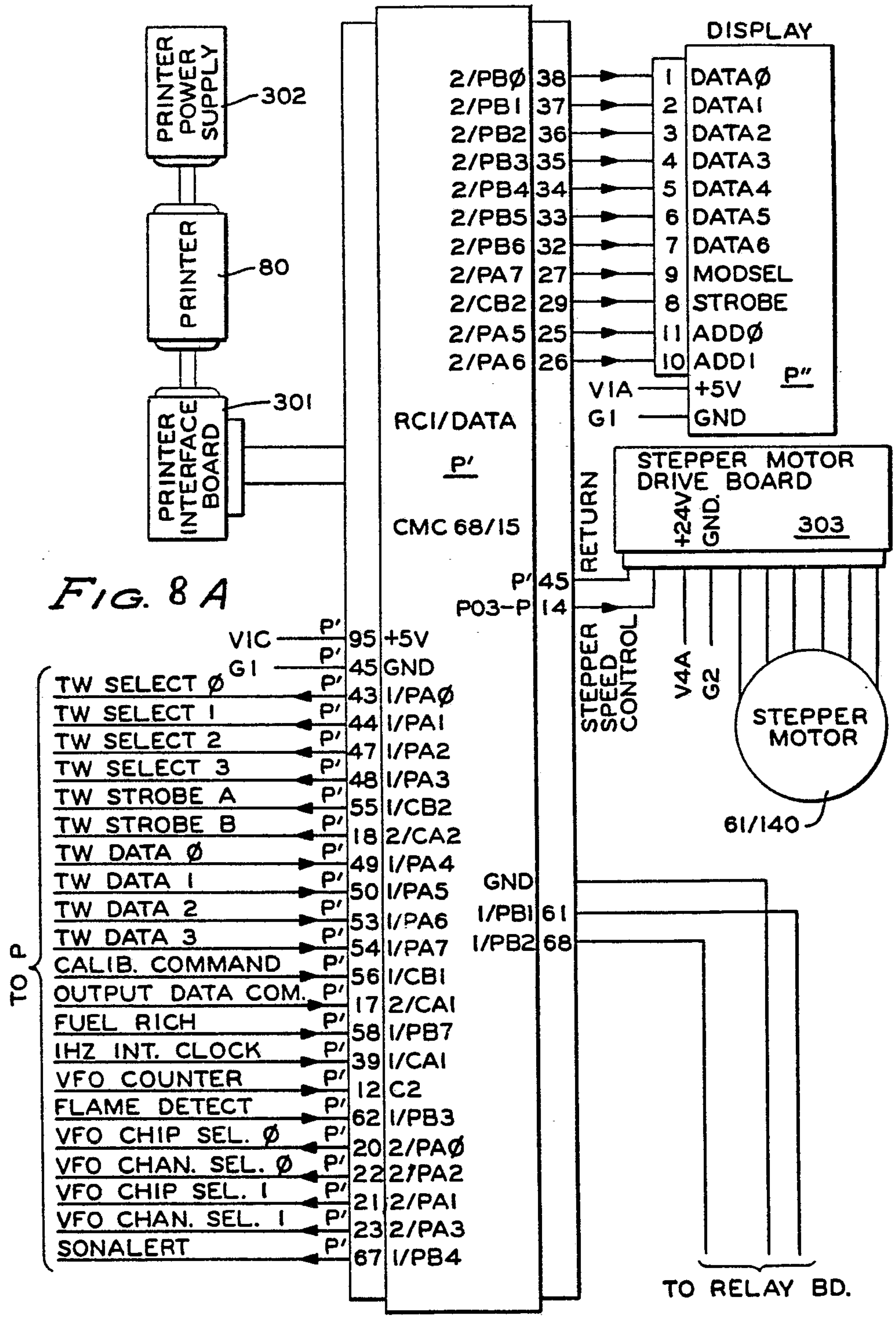

PRINTER POWER SUPPLY
302
PRINTER
80
PRINTER INTERFACE BOARD
301
DISPLAY
2/PBØ 38
2/PB1 37
2/PB2 36
2/PB3 35
2/PB4 34
2/PB5 33
2/PB6 32
2/PA7 27
2/CB2 29
2/PA5 25
2/PA6 26
1 DATAØ
2 DATA1
3 DATA2
4 DATA3
5 DATA4
6 DATA5
7 DATA6
9 MODSEL
8 STROBE
11 ADDØ
10 ADD1
V1A +5V
G1 GND
P″
RC1/DATA
P′
CMC 68/15
RETURN
STEPPER MOTOR DRIVE BOARD
+24V
GND.
303
P′ 45
P03-P 14
STEPPER SPEED CONTROL
V4A
G2
STEPPER MOTOR
61/140
V1C P′ 95 +5V
G1 P′ 45 GND
TW SELECT Ø P′ 43 1/PAØ
TW SELECT 1 P′ 44 1/PA1
TW SELECT 2 P′ 47 1/PA2
TW SELECT 3 P′ 48 1/PA3
TW STROBE A P′ 55 1/CB2
TW STROBE B P′ 18 2/CA2
TW DATA Ø P′ 49 1/PA4
TW DATA 1 P′ 50 1/PA5
TW DATA 2 P′ 53 1/PA6
TW DATA 3 P′ 54 1/PA7
CALIB. COMMAND P′ 56 1/CB1
OUTPUT DATA COM. P′ 17 2/CA1
FUEL RICH P′ 58 1/PB7
1HZ INT. CLOCK P′ 39 1/CA1
VFO COUNTER P′ 12 C2
FLAME DETECT P′ 62 1/PB3
VFO CHIP SEL. Ø P′ 20 2/PAØ
VFO CHAN. SEL. Ø P′ 22 2/PA2
VFO CHIP SEL. 1 P′ 21 2/PA1
VFO CHAN. SEL. 1 P′ 23 2/PA3
SONALERT P′ 67 1/PB4
TO P
GND
1/PB1 61
1/PB2 68
TO RELAY BD.


FIG. 8A

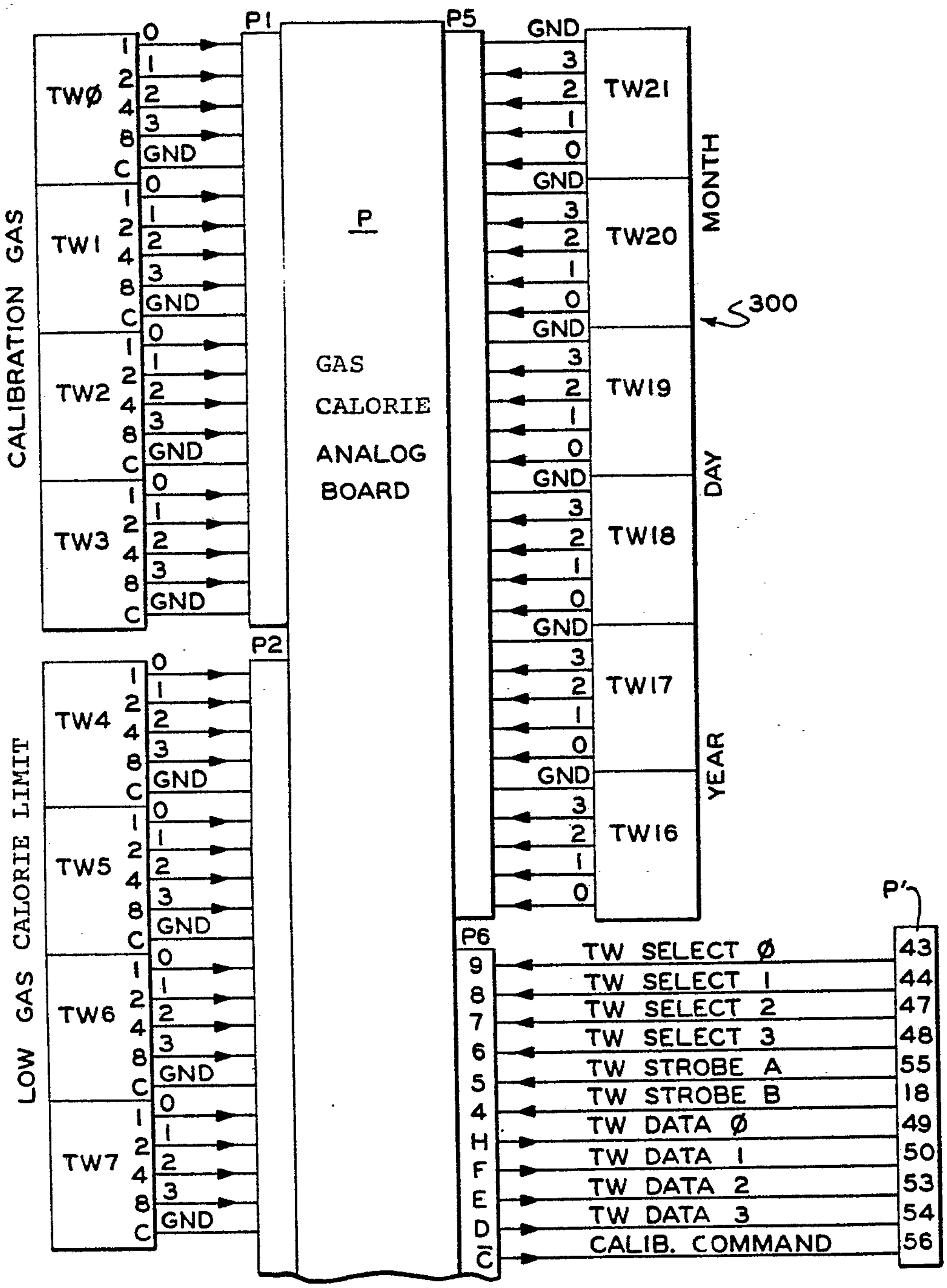
P1
P5
P2
P6
P
GAS
CALORIE
ANALOG
BOARD
TWØ
TW1
TW2
TW3
TW4
TW5
TW6
TW7
CALIBRATION GAS
LOW GAS CALORIE LIMIT
TW21
TW20
TW19
TW18
TW17
TW16
MONTH
DAY
YEAR
300
P'
TW SELECT Ø 43
TW SELECT 1 44
TW SELECT 2 47
TW SELECT 3 48
TW STROBE A 55
TW STROBE B 18
TW DATA Ø 49
TW DATA 1 50
TW DATA 2 53
TW DATA 3 54
CALIB. COMMAND 56
GND

FIG. 8B

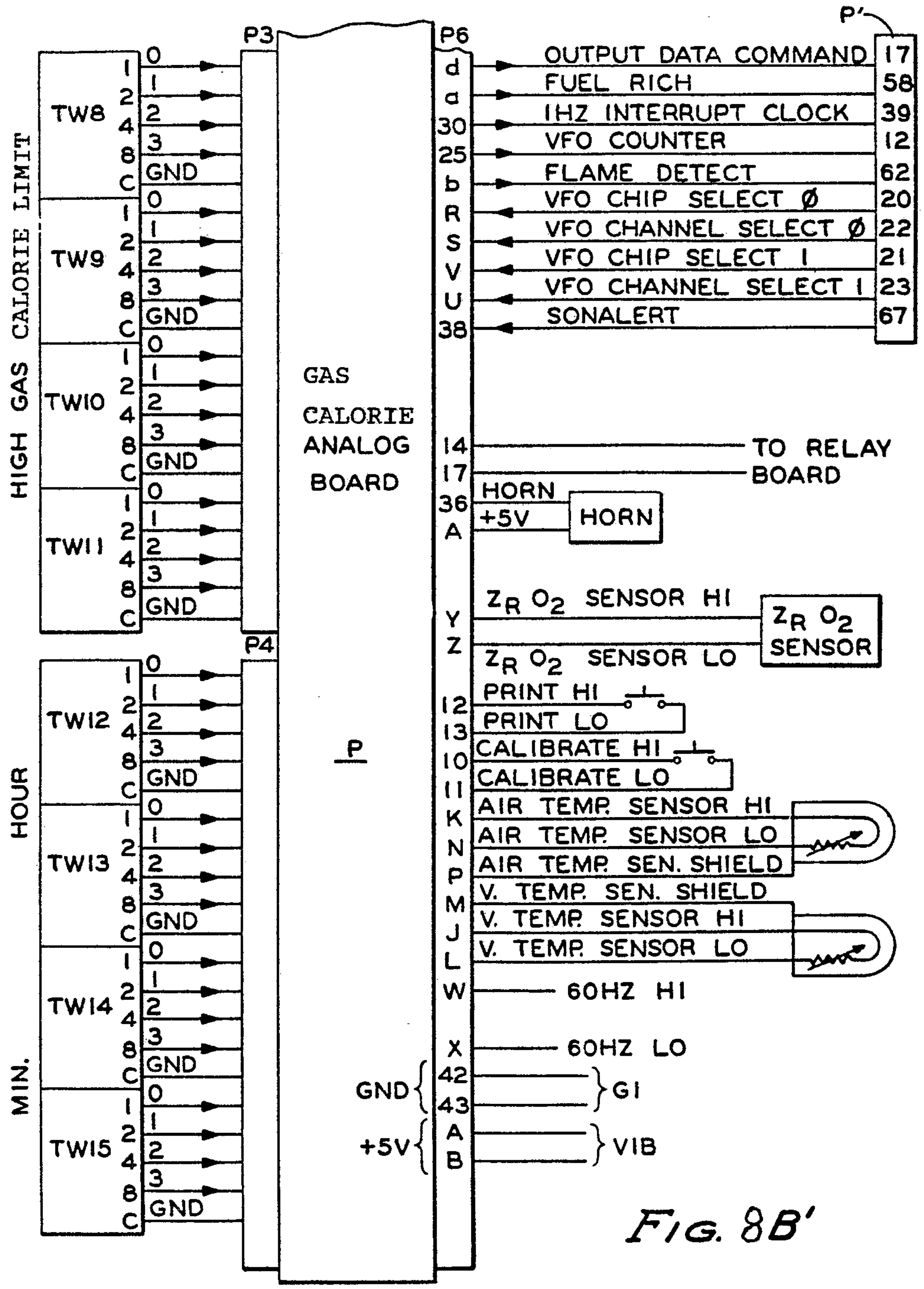
P'
P3
P6
P4
TW8
TW9
TW10
TW11
TW12
TW13
TW14
TW15
HIGH GAS CALORIE LIMIT
HOUR
MIN.
GND
GAS CALORIE ANALOG BOARD
P
OUTPUT DATA COMMAND 17
FUEL RICH 58
1HZ INTERRUPT CLOCK 39
VFO COUNTER 12
FLAME DETECT 62
VFO CHIP SELECT Ø 20
VFO CHANNEL SELECT Ø 22
VFO CHIP SELECT 1 21
VFO CHANNEL SELECT 1 23
SONALERT 67
TO RELAY BOARD
HORN
+5V
HORN
ZR O2 SENSOR HI
ZR O2 SENSOR
ZR O2 SENSOR LO
PRINT HI
PRINT LO
CALIBRATE HI
CALIBRATE LO
AIR TEMP. SENSOR HI
AIR TEMP. SENSOR LO
AIR TEMP. SEN. SHIELD
V. TEMP. SEN. SHIELD
V. TEMP. SENSOR HI
V. TEMP. SENSOR LO
60HZ HI
60HZ LO
GND
+5V
G1
V1B

FIG. 8B'

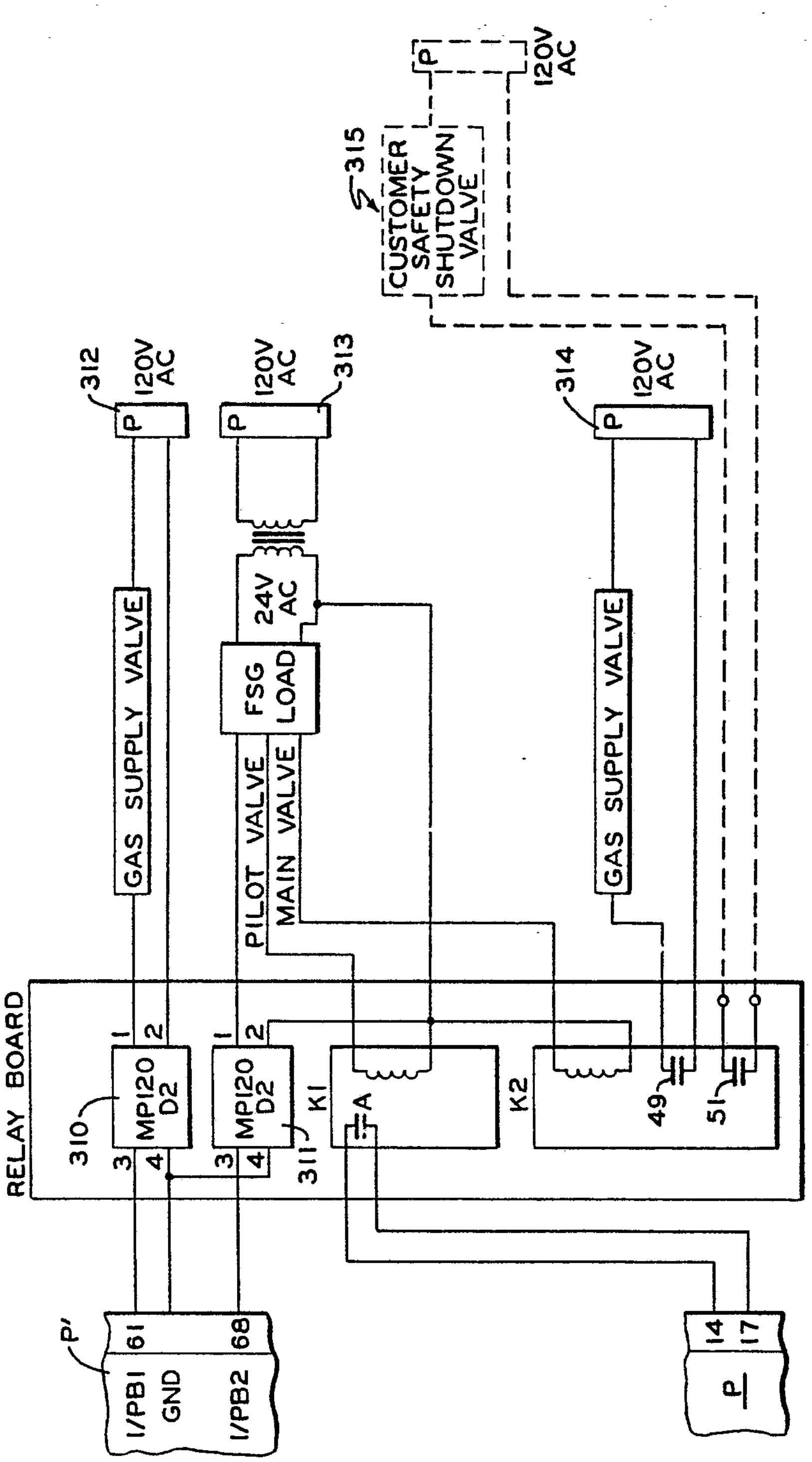
RELAY BOARD
310
MP120 D2
311
K1
A
K2
49
51
1
2
3
4
P'
I/PB1
GND
I/PB2
61
68
P
14
17
GAS SUPPLY VALVE
PILOT VALVE
MAIN VALVE
FSG LOAD
24V AC
312
313
314
P
120V AC
315
CUSTOMER SAFETY SHUTDOWN VALVE

FIG. 8C

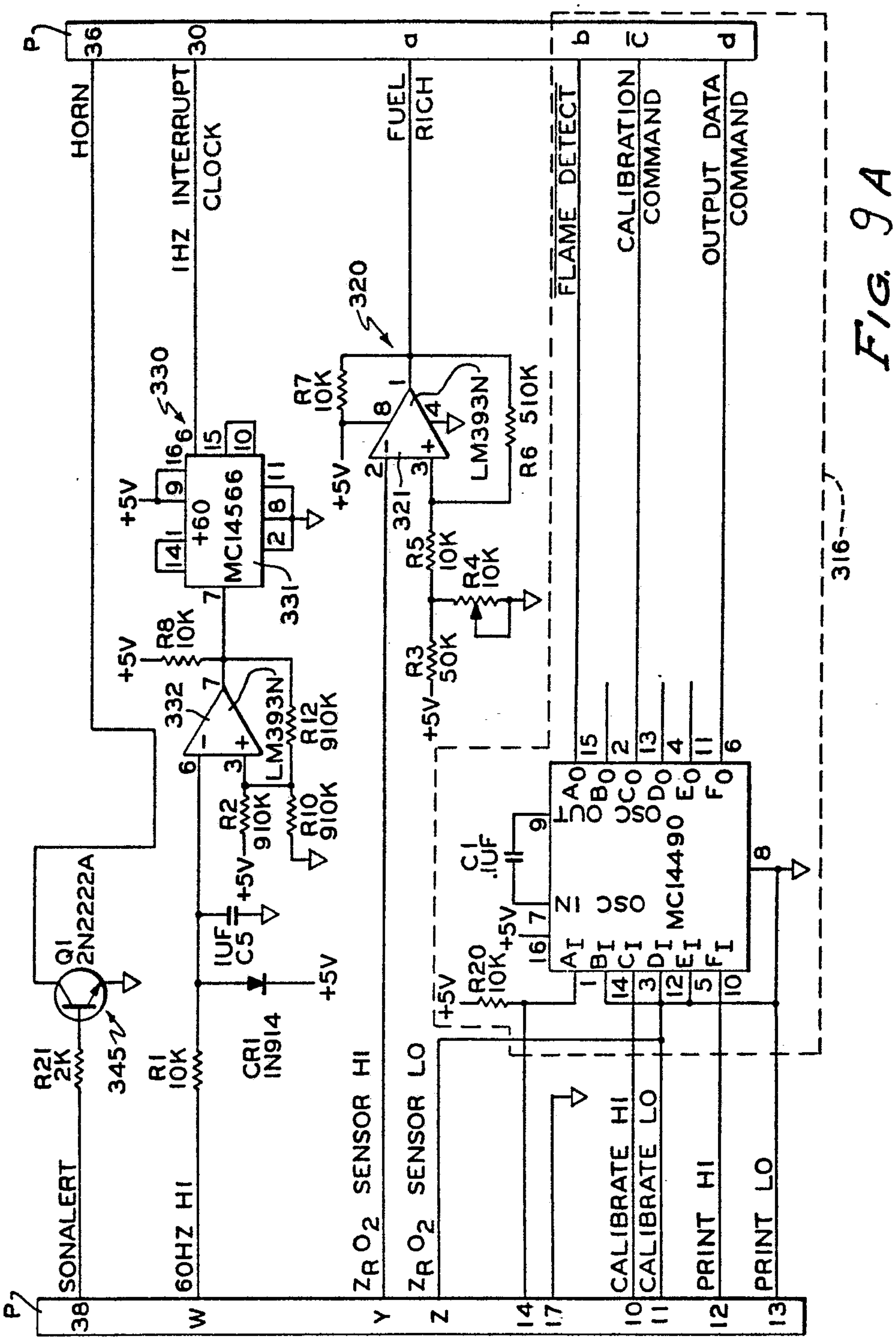
P
36
30
a
b
c̄
d
HORN
1HZ INTERRUPT
CLOCK
FUEL
RICH
FLAME DETECT
CALIBRATION
COMMAND
OUTPUT DATA
COMMAND
316
320
330
331
332
321
345
+60
MC14566
LM393N
MC14490
Q1
2N2222A
R21
2K
R1
10K
CR1
1N914
1UF
C5
R2
910K
R10
910K
R12
910K
R8
10K
R7
10K
R6 510K
R5
10K
R4
10K
R3
50K
R20
10K
C1
.1UF
+5V
SONALERT
60HZ HI
$Z_RO_2$ SENSOR HI
$Z_RO_2$ SENSOR LO
CALIBRATE HI
CALIBRATE LO
PRINT HI
PRINT LO
38
W
Y
Z
14
17
10
11
12
13

FIG. 9A

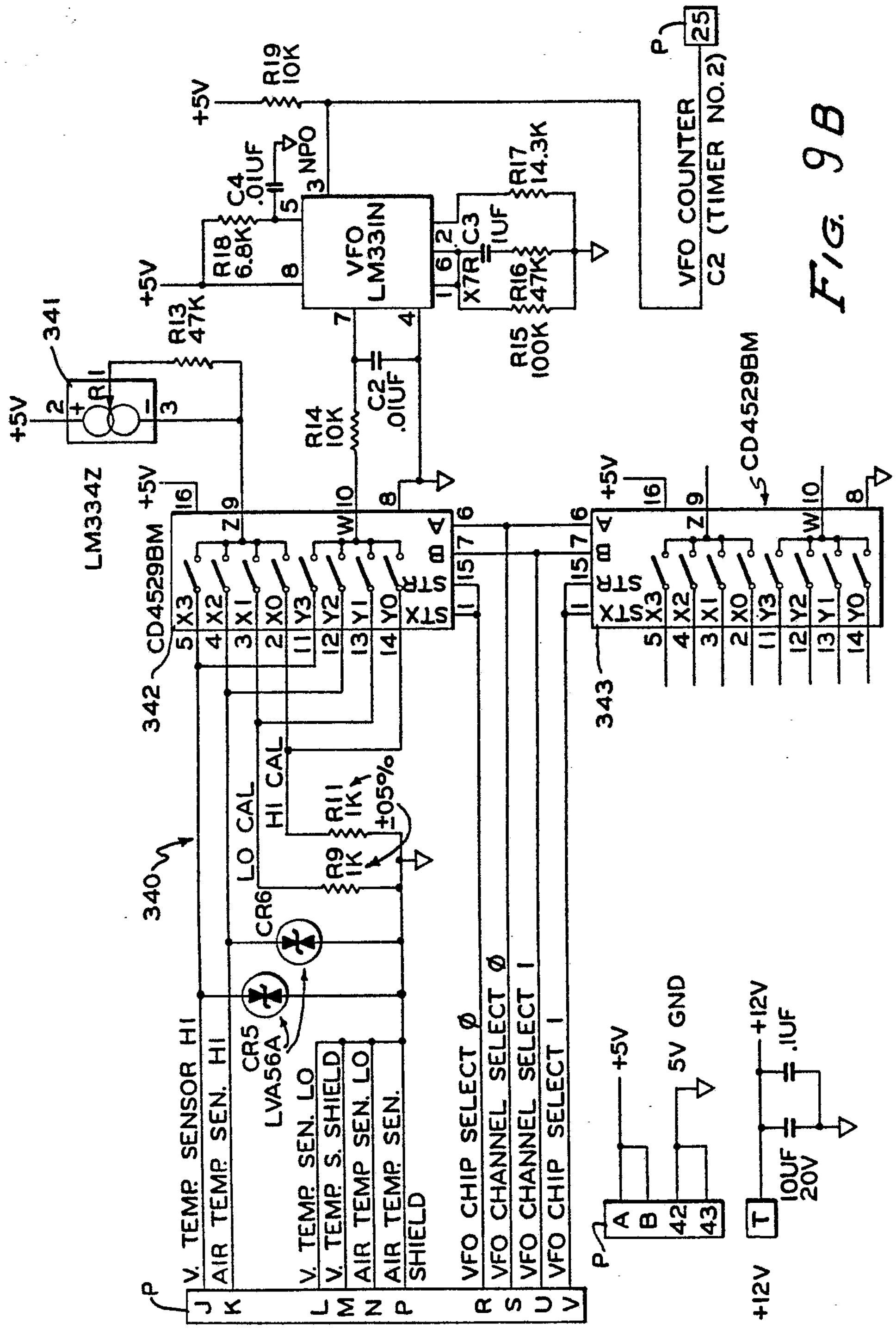

+5V
R19 10K
C4 .01UF
NPO
R18 6.8K
VFO LM331N
C3 .1UF
X7R
R17 14.3K
R16 47K
R15 100K
C2 .01UF
R14 10K
R13 47K
341
R1
LM334Z
CD4529BM
VFO COUNTER
C2 (TIMER NO.2)
P
25
342
343
340
LO CAL
HI CAL
R9 1K
R11 1K
±.05%
CR6
CR5
LVA56A
V. TEMP. SENSOR HI
AIR TEMP. SEN. HI
V. TEMP. SEN. LO
V. TEMP. S. SHIELD
AIR TEMP. SEN. LO
AIR TEMP. SEN. SHIELD
VFO CHIP SELECT Ø
VFO CHANNEL SELECT Ø
VFO CHANNEL SELECT 1
VFO CHIP SELECT 1
5V GND
+12V
10UF 20V
.1UF


FIG. 9B

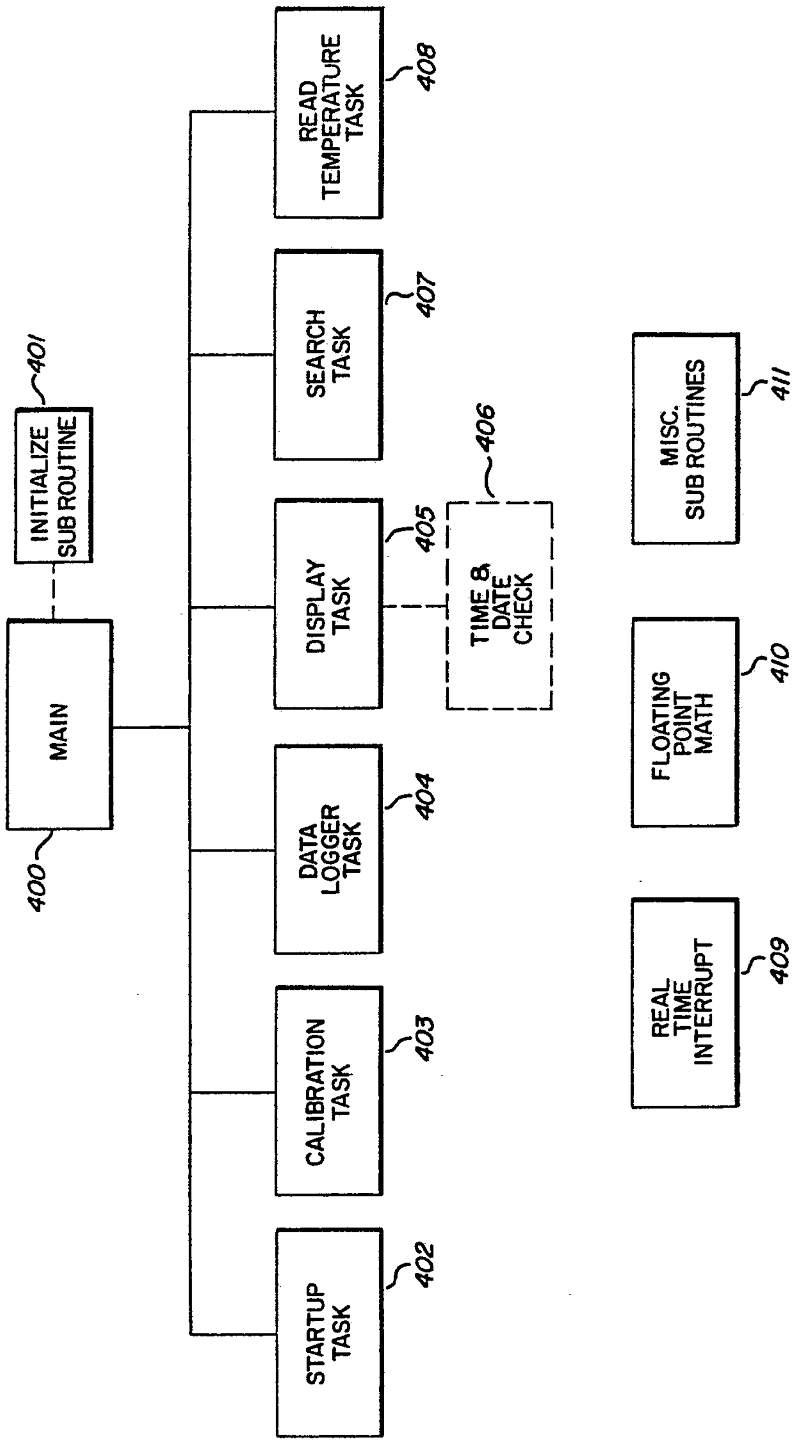
MAIN
400
INITIALIZE SUB ROUTINE
401
STARTUP TASK
402
CALIBRATION TASK
403
DATA LOGGER TASK
404
DISPLAY TASK
405
TIME & DATE CHECK
406
SEARCH TASK
407
READ TEMPERATURE TASK
408
REAL TIME INTERRUPT
409
FLOATING POINT MATH
410
MISC. SUB ROUTINES
411

FIG. 10

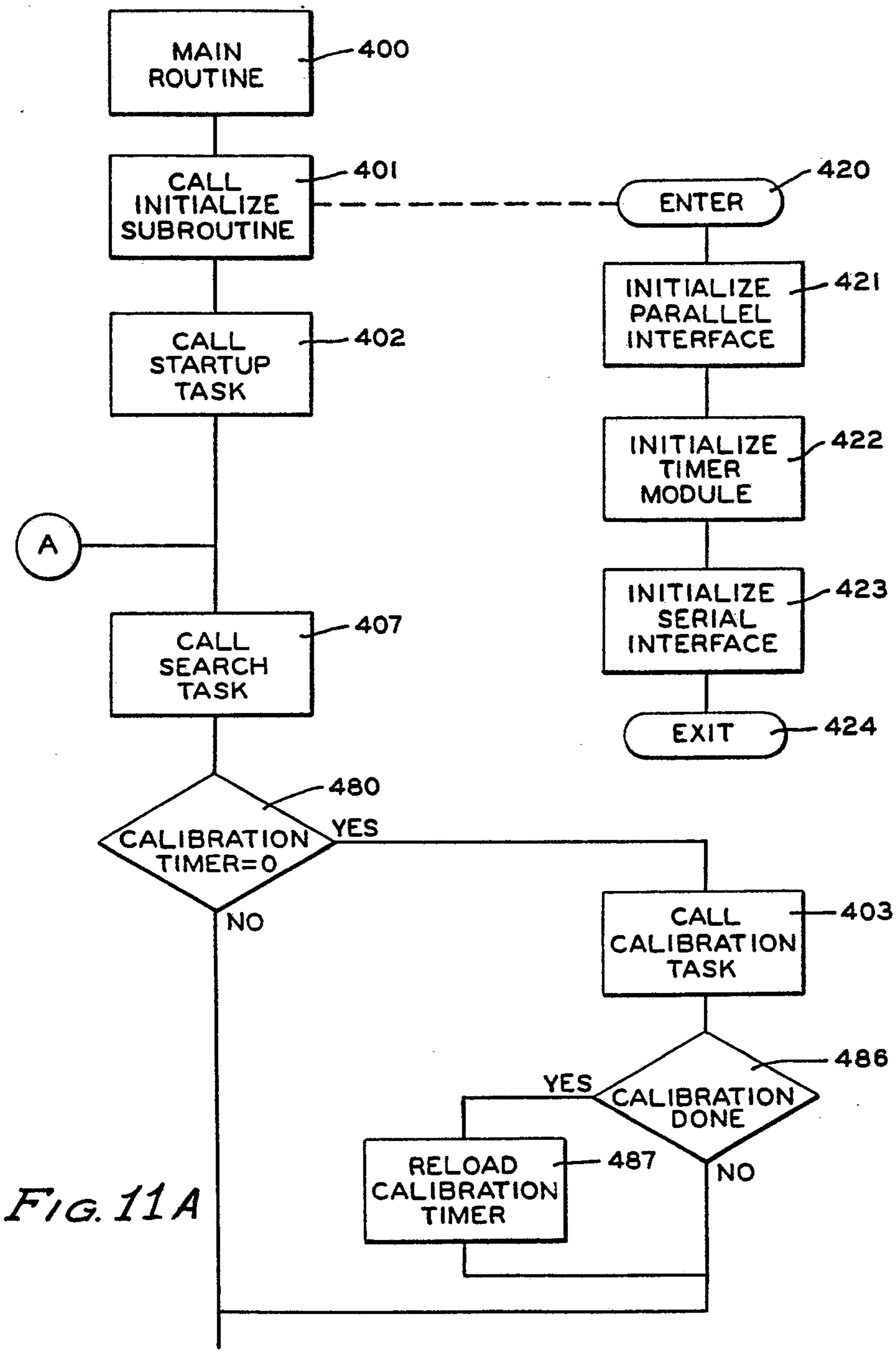
MAIN ROUTINE
400
CALL INITIALIZE SUBROUTINE
401
ENTER
420
INITIALIZE PARALLEL INTERFACE
421
INITIALIZE TIMER MODULE
422
INITIALIZE SERIAL INTERFACE
423
EXIT
424
CALL STARTUP TASK
402
A
CALL SEARCH TASK
407
480
CALIBRATION TIMER=0
YES
NO
CALL CALIBRATION TASK
403
486
CALIBRATION DONE
YES
NO
RELOAD CALIBRATION TIMER
487

FIG. 11A

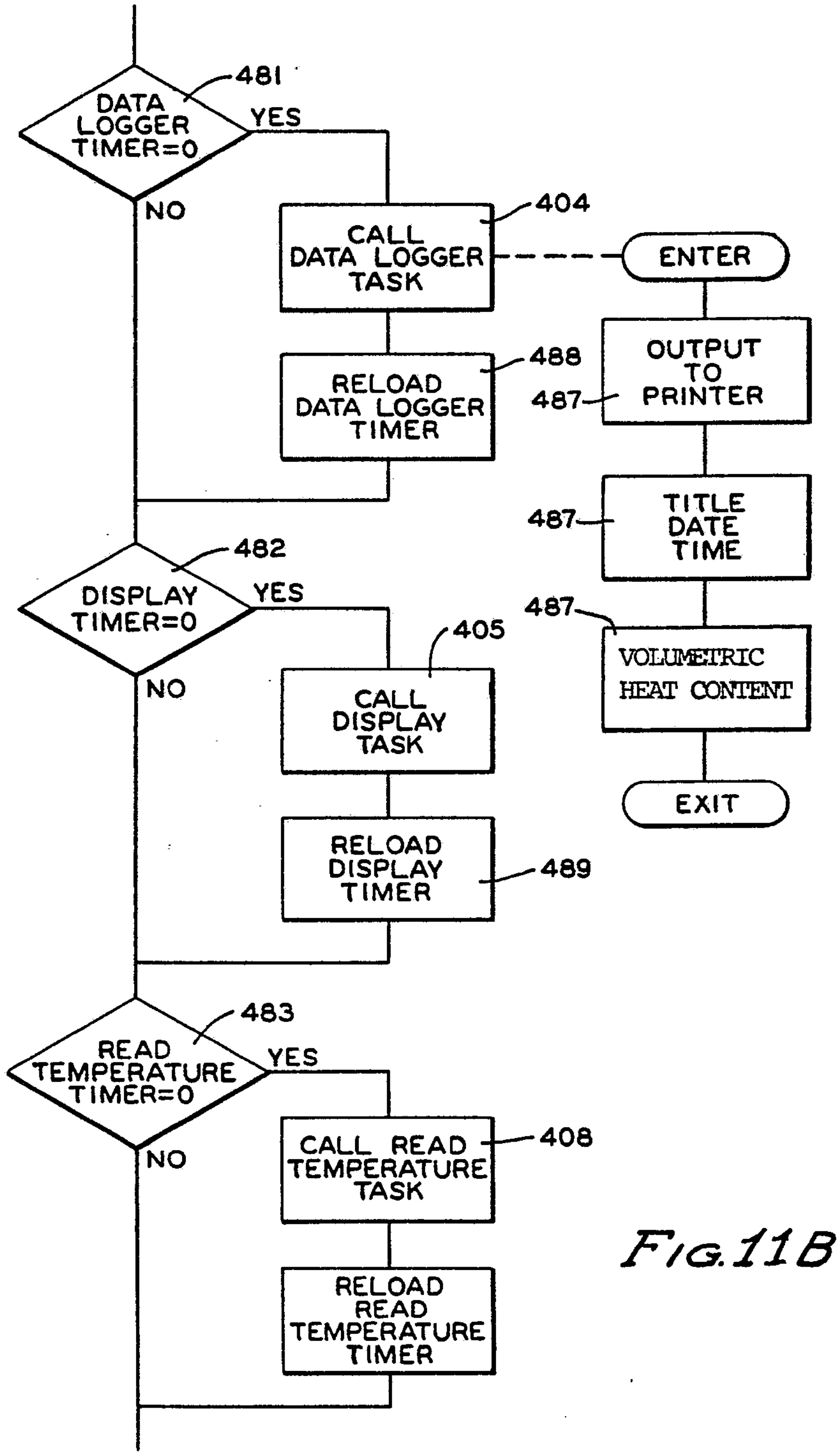
DATA LOGGER TIMER=0
481
YES
NO
CALL DATA LOGGER TASK
404
ENTER
OUTPUT TO PRINTER
487
RELOAD DATA LOGGER TIMER
488
TITLE DATE TIME
487
DISPLAY TIMER=0
482
YES
NO
487
VOLUMETRIC HEAT CONTENT
CALL DISPLAY TASK
405
EXIT
RELOAD DISPLAY TIMER
489
READ TEMPERATURE TIMER=0
483
YES
NO
CALL READ TEMPERATURE TASK
408
RELOAD READ TEMPERATURE TIMER

FIG. 11B

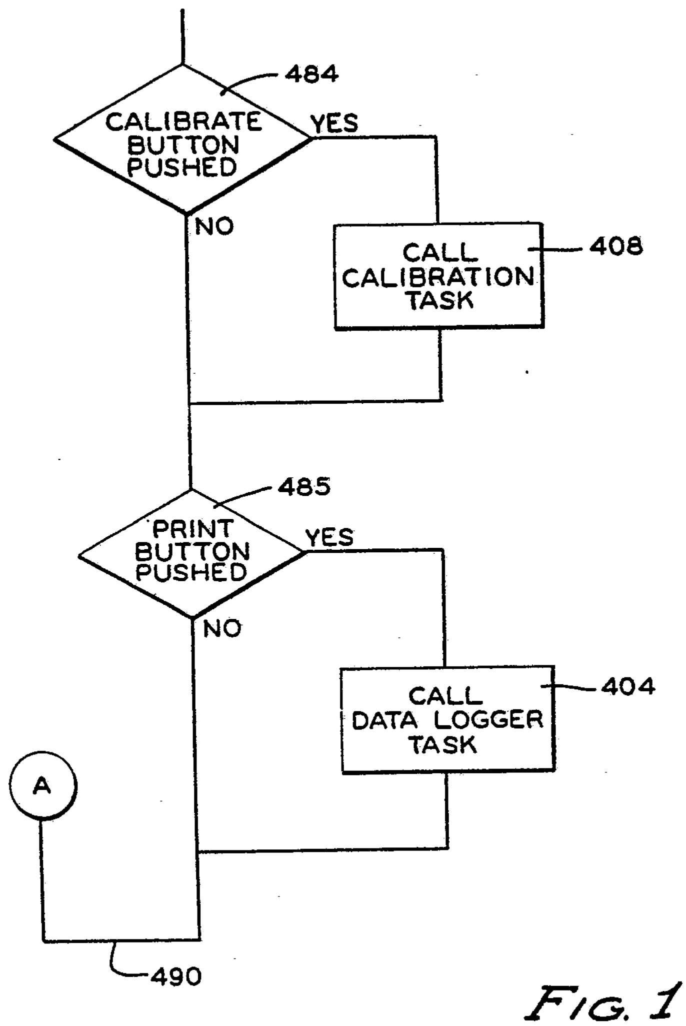
484
CALIBRATE BUTTON PUSHED
YES
NO
CALL CALIBRATION TASK
408
485
PRINT BUTTON PUSHED
YES
NO
CALL DATA LOGGER TASK
404
A
490

FIG. 11C

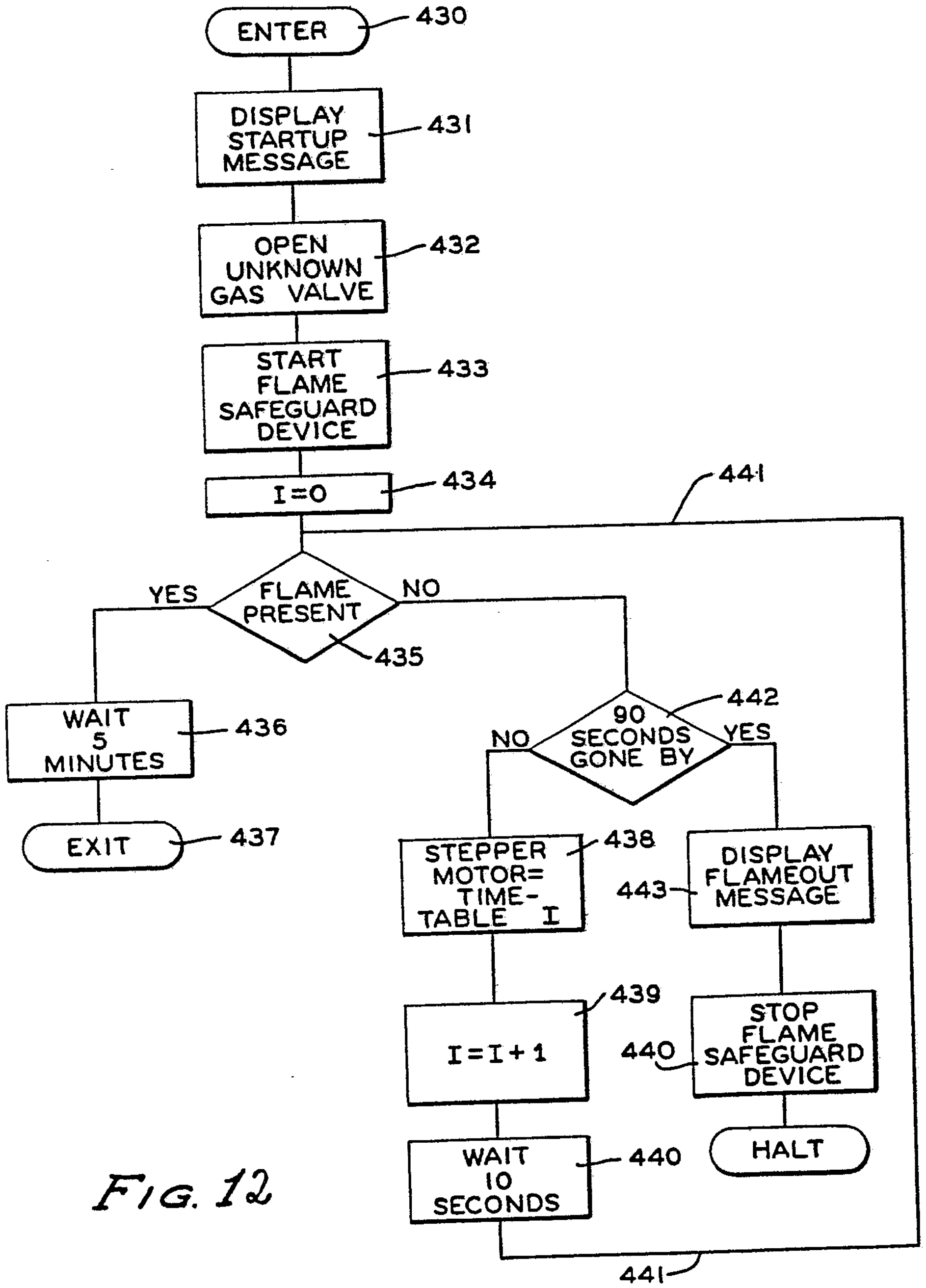
ENTER
430
DISPLAY STARTUP MESSAGE
431
OPEN UNKNOWN GAS VALVE
432
START FLAME SAFEGUARD DEVICE
433
I=0
434
441
FLAME PRESENT
YES
NO
435
WAIT 5 MINUTES
436
EXIT
437
90 SECONDS GONE BY
442
NO
YES
STEPPER MOTOR= TIME-TABLE I
438
DISPLAY FLAMEOUT MESSAGE
443
I=I+1
439
STOP FLAME SAFEGUARD DEVICE
440
HALT
WAIT 10 SECONDS
440
441

FIG. 12

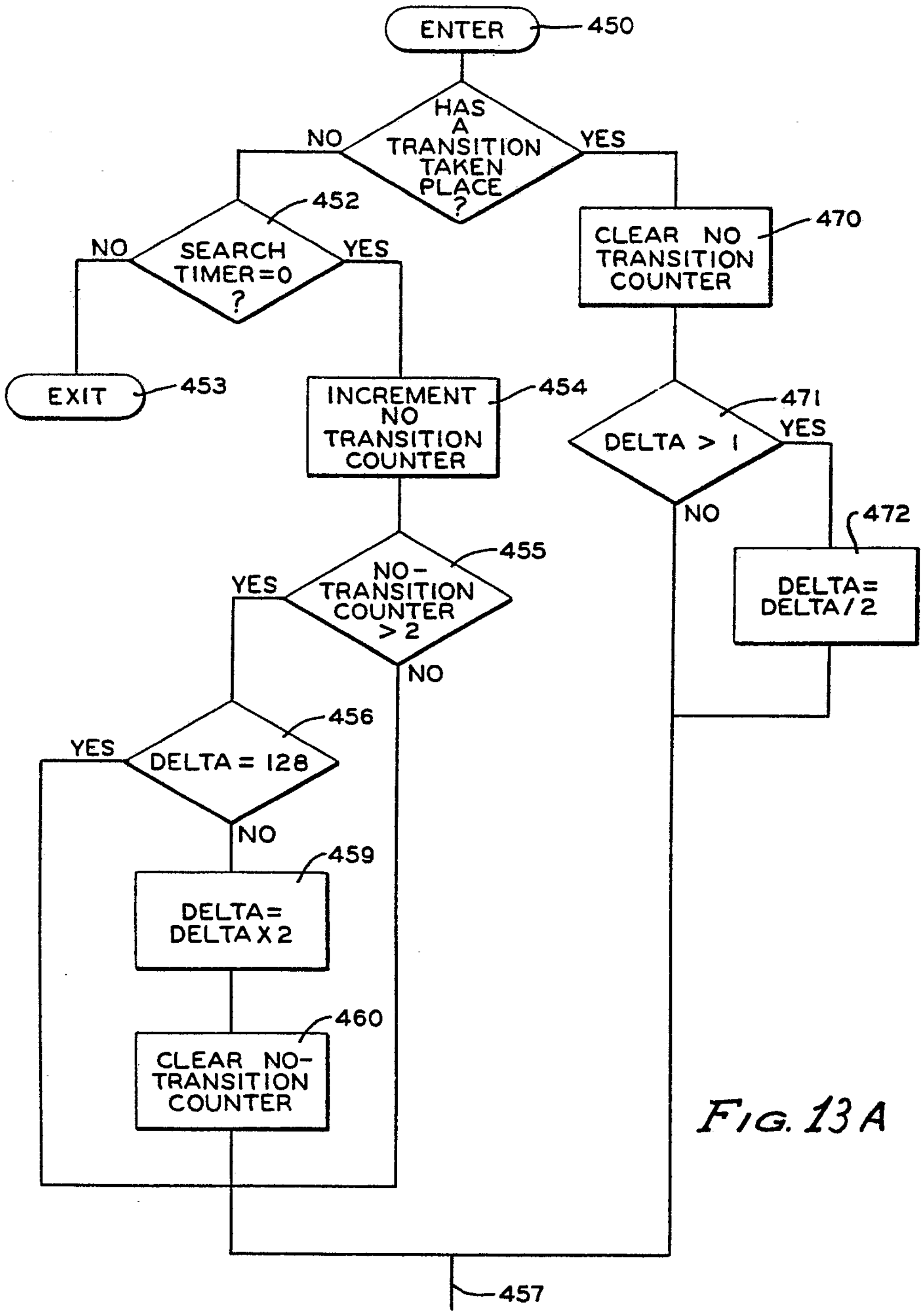
ENTER
450
HAS A TRANSITION TAKEN PLACE ?
NO
YES
452
SEARCH TIMER=0 ?
NO
YES
470
CLEAR NO TRANSITION COUNTER
EXIT
453
INCREMENT NO TRANSITION COUNTER
454
471
DELTA > 1
YES
NO
472
DELTA= DELTA/2
455
NO-TRANSITION COUNTER > 2
YES
NO
456
DELTA = 128
YES
NO
459
DELTA= DELTA X 2
460
CLEAR NO-TRANSITION COUNTER
457

*Fig. 13 A*

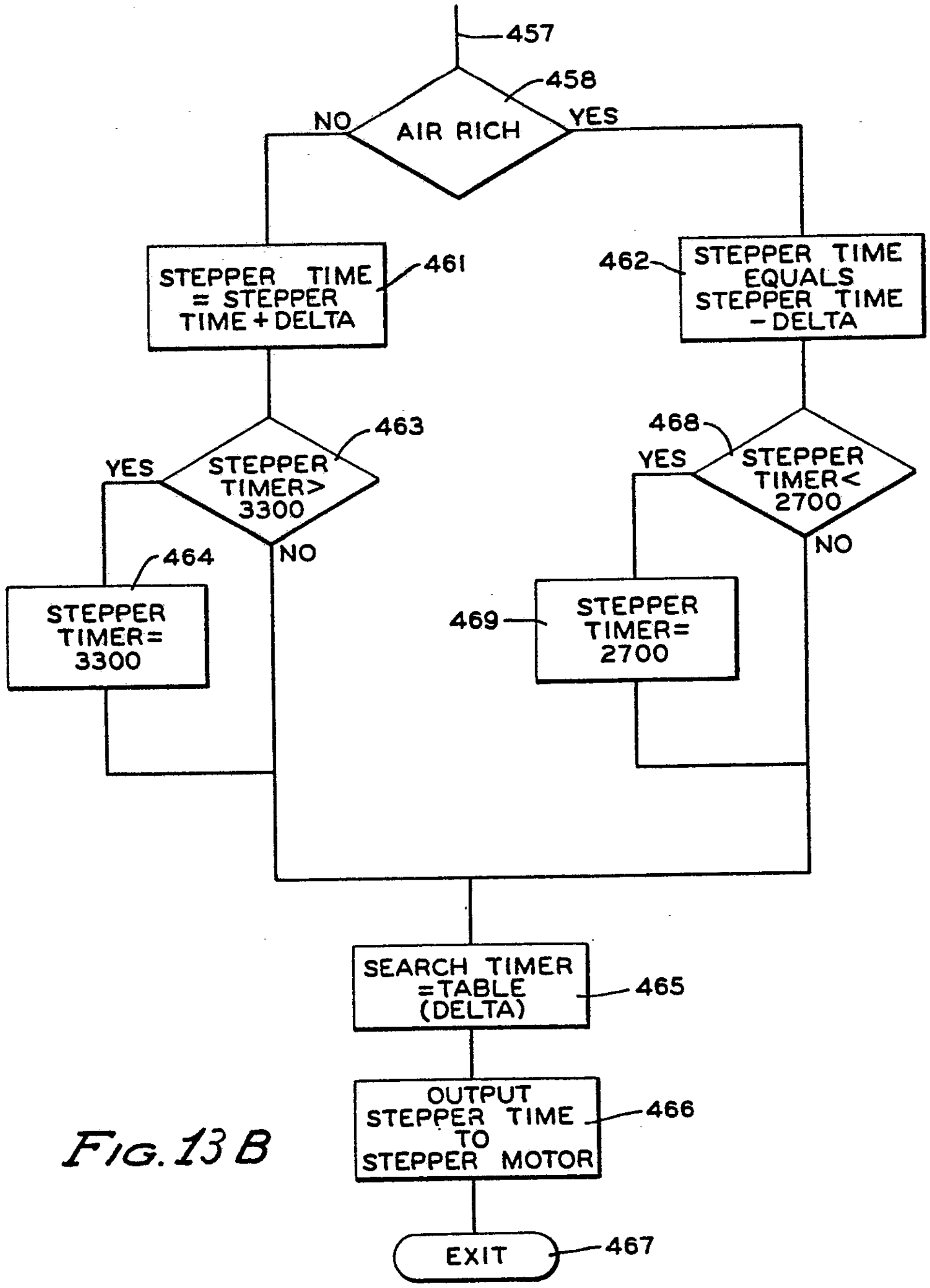
457
458
AIR RICH
NO
YES
STEPPER TIME = STEPPER TIME + DELTA
461
462
STEPPER TIME EQUALS STEPPER TIME - DELTA
463
YES
STEPPER TIMER> 3300
NO
464
STEPPER TIMER= 3300
468
YES
STEPPER TIMER< 2700
NO
469
STEPPER TIMER= 2700
SEARCH TIMER =TABLE (DELTA)
465
OUTPUT STEPPER TIME TO STEPPER MOTOR
466
EXIT
467

FIG. 13 B

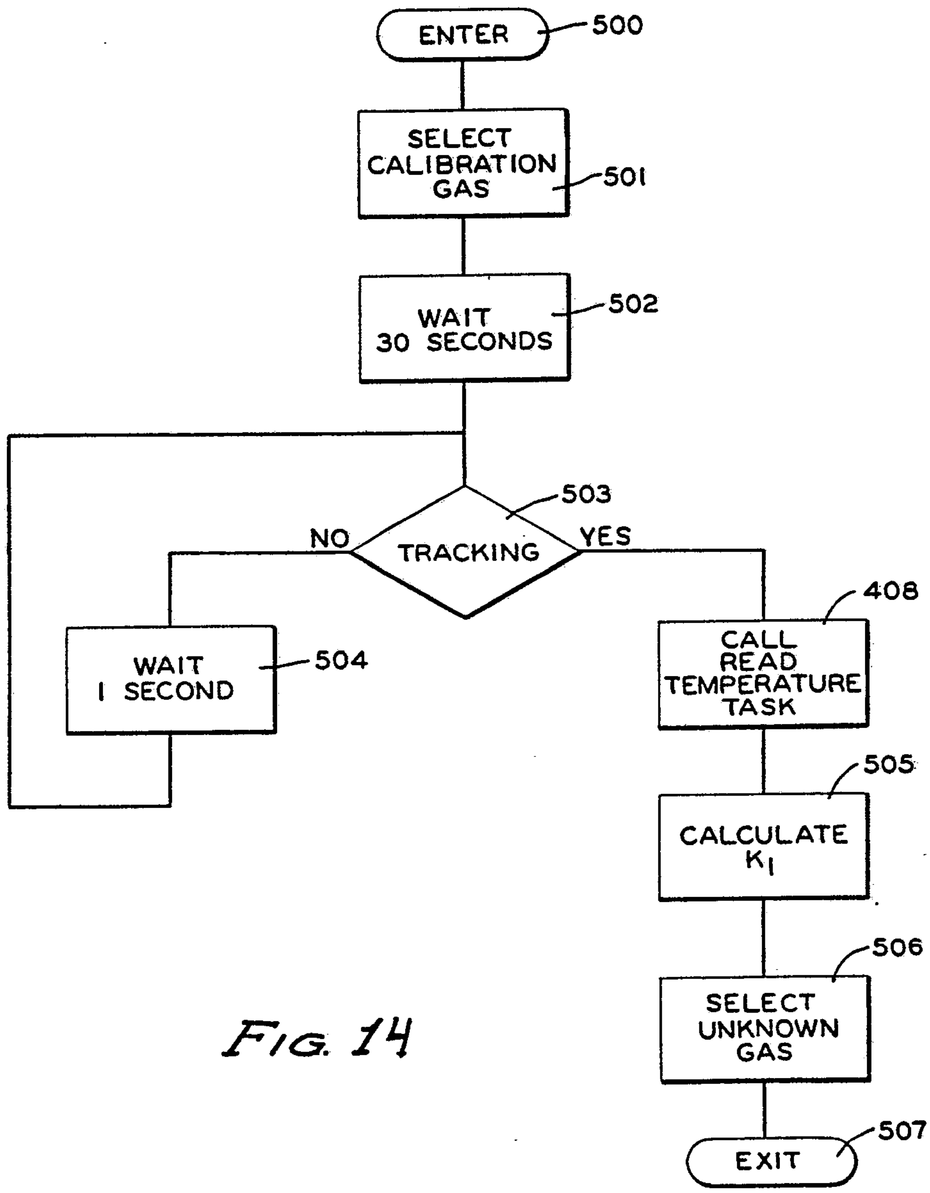
ENTER
500
SELECT CALIBRATION GAS
501
WAIT 30 SECONDS
502
503
NO
TRACKING
YES
WAIT 1 SECOND
504
408
CALL READ TEMPERATURE TASK
505
CALCULATE $K_1$
506
SELECT UNKNOWN GAS
507
EXIT

FIG. 14

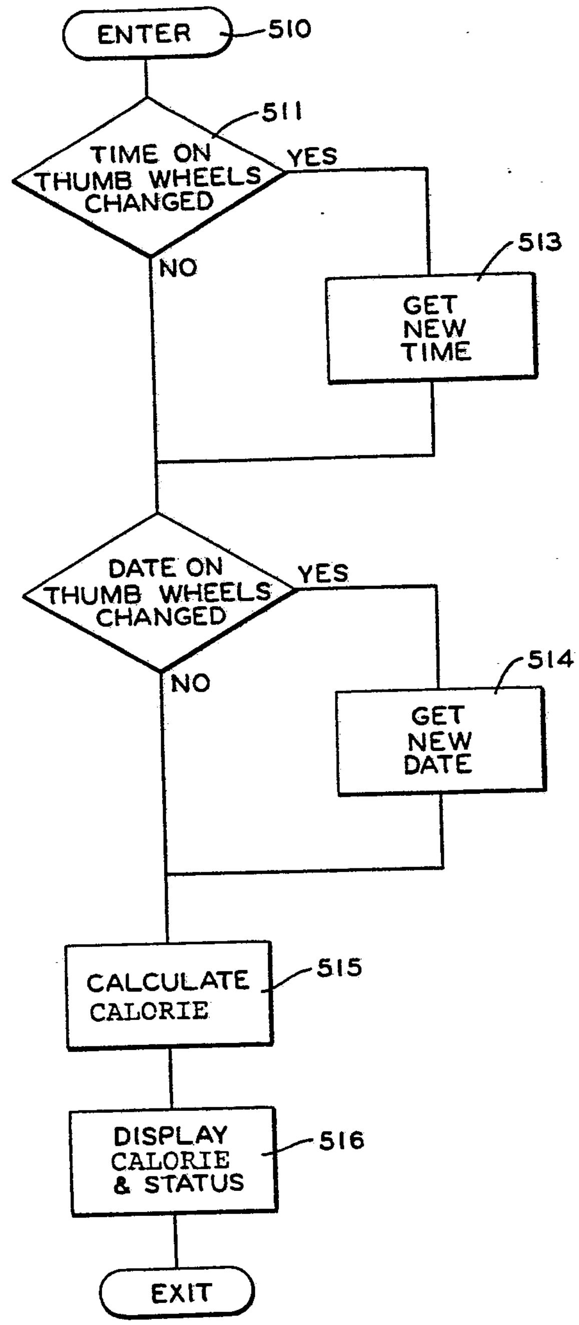
ENTER
510
511
TIME ON THUMB WHEELS CHANGED
YES
NO
513
GET NEW TIME
DATE ON THUMB WHEELS CHANGED
YES
NO
514
GET NEW DATE
CALCULATE CALORIE
515
DISPLAY CALORIE & STATUS
516
EXIT

FIG. 15

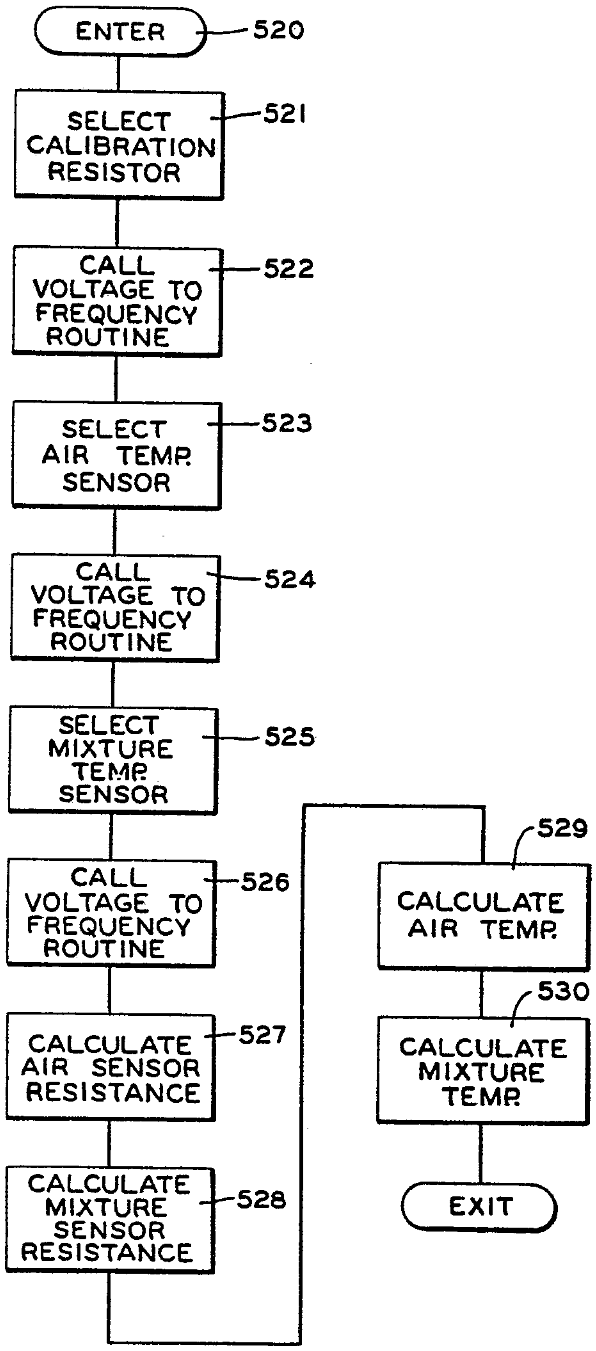
ENTER
520
SELECT CALIBRATION RESISTOR
521
CALL VOLTAGE TO FREQUENCY ROUTINE
522
SELECT AIR TEMP. SENSOR
523
CALL VOLTAGE TO FREQUENCY ROUTINE
524
SELECT MIXTURE TEMP. SENSOR
525
CALL VOLTAGE TO FREQUENCY ROUTINE
526
CALCULATE AIR SENSOR RESISTANCE
527
CALCULATE MIXTURE SENSOR RESISTANCE
528
CALCULATE AIR TEMP.
529
CALCULATE MIXTURE TEMP.
530
EXIT

FIG. 16

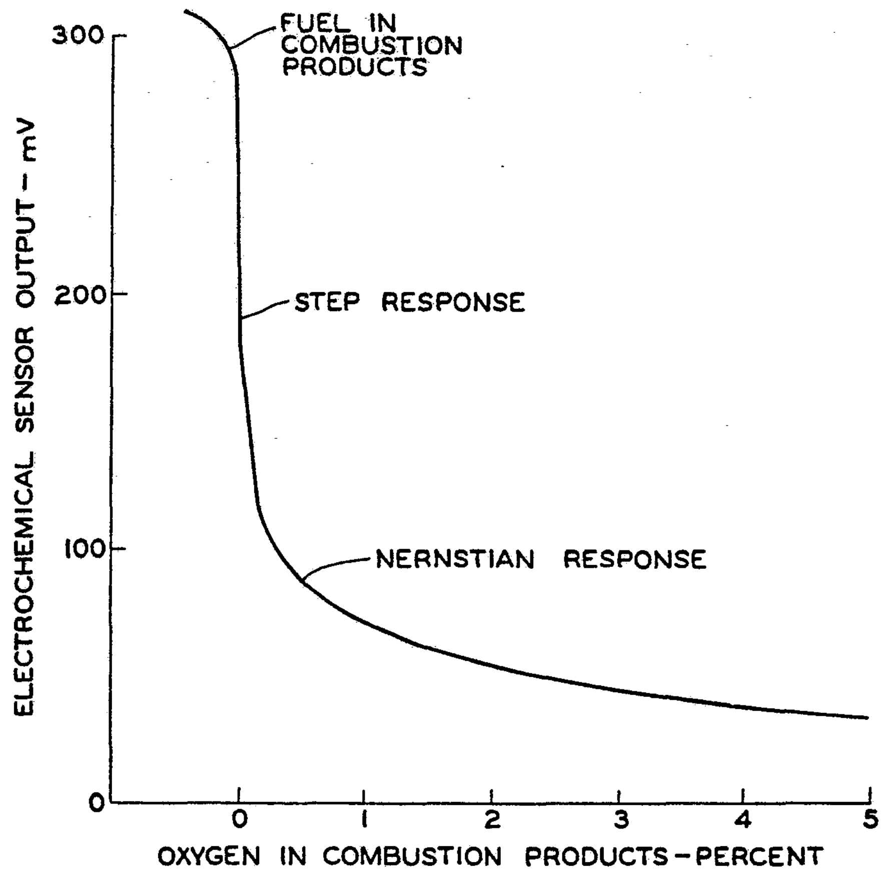


TYPICAL ELECTROCHEMICAL SENSOR RESPONSE

FIG. 17

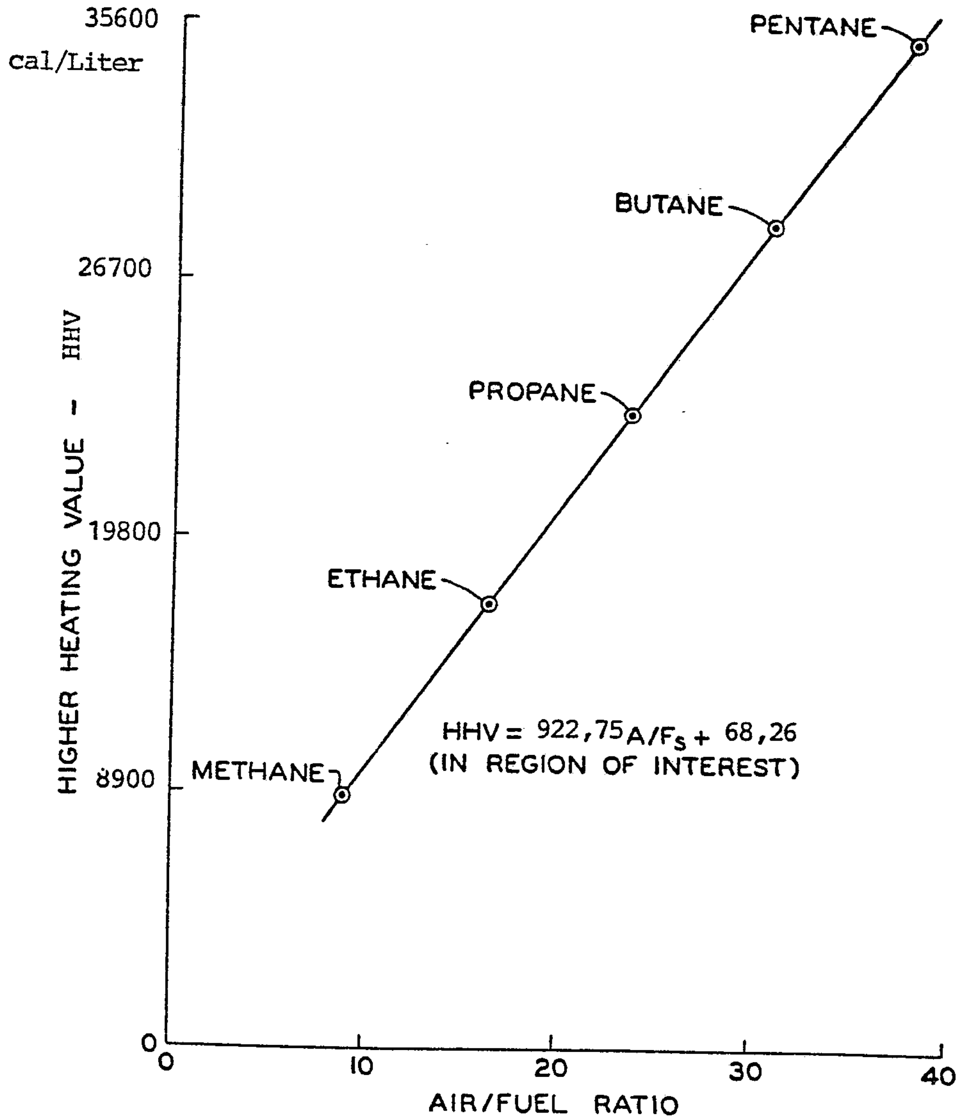
35600
cal/Liter
26700
19800
8900
0
HIGHER HEATING VALUE - HHV
PENTANE
BUTANE
PROPANE
ETHANE
METHANE
HHV = 922,75A/Fs + 68,26
(IN REGION OF INTEREST)
0
10
20
30
40
AIR/FUEL RATIO

FIG. 18